# EUROPEAN PATENT APPLICATION

(11) **EP 1 186 293 A2**
(43) Date of publication of application: **13.03.2002**
(21) Application number: 01307229.3
(22) Date of filing: 24.08.2001
(51) Int. Cl.: A61K 31/00, A61K 31/437, A61K 31/444, A61P 3/04, A61P 3/10, A61P 9/04, A61P 19/00, A61P 19/10

(54) **Intermittent administration of a growth hormone secretagogue**

(30) Priority: 30.08.2000 US 229077 P
(71) Applicant: Pfizer Products Inc., Groton, Connecticut 06340 (US)
(72) Inventor: Maclean, David Burton, Pfizer Global, Groton, Connecticut 06340 (US)
(74) Representative: Ruddock, Keith Stephen

(57) **Abstract**

The present invention relates to the intermittent administration of a growth hormone secretagogue to a patient and to kits for use therein.

## Description

### Field of the Invention

The present invention relates to methods of administering a growth hormone secretagogue to a patient. In particular, the methods comprise the intermittent administration of a growth hormone secretagogue to a patient.

### Background of the Invention

Growth hormone (GH), which is secreted by the pituitary gland, stimulates the growth of all tissues of the body that are capable of growing. In addition, growth hormone is known to have the following effects on metabolic processes:
1. increased rate of protein synthesis in substantially all cells;
2. decreased rate of carbohydrate metabolism in cells; and
3. increased mobilization of free fatty acids and use of fatty acids for energy.

A deficiency in GH production and/or secretion can result in various diseases or conditions, such as dwarfism, profound reduction in lean body mass and concomitant increase in total body fat, particularly in the truncal region, decreased skeletal and cardiac muscle mass and muscle strength that can result in significant decreases in exercise capacity, musculoskeletal frailty, which is typically associated with old age, congestive heart failure, insulin resistance, bone fracture, reduction in bone density, delayed wound healing, and osteoporosis. The administration of exogenous growth hormone has been shown to reverse the above-mentioned metabolic changes and has also been shown to lower plasma low density lipoprotein (LDL) cholesterol and improve psychological well being.

With the rapid worldwide growth of the population aged 65 years and over, aging-associated musculoskeletal frailty will become an increasing public health problem. Frailty, in addition to its personal impact on daily functioning and social interaction, is associated with major health consequences such as injurious falls, hip fractures, and nursing home admissions. Annually, in the United States, up to 10% of frail adults over age 74 experience an injurious fall.

The causes of the long term age-associated decline in muscle and bone mass, which after age 40 in both men and women averages 0.5-1% per year, are unknown. A decline in synthesis/secretion of endogenous anabolic hormones may contribute to age-associated changes in body composition, which are characterized by decreased muscle and bone mass and a relative increase in adiposity. For example, in both men and women, growth hormone (GH, also termed somatotropin) secretion declines by 50% between the ages of 30 and 70.

GH is naturally released by the body in a patterned manner with typically large pulses during sleep and subsequent smaller pulses of GH released later. It is also believed that growth hormone releasing hormone [GHRH, also known as growth hormone releasing factor (GRF)] is released from the hypothalamus in a pulsatile or patterned manner and consequently stimulates the release of GH in a correspondingly patterned manner. Other factors including somatostatin and growth hormone releasing related peptides also contribute to the pulse frequency or peak height of growth hormone secretion.

In cases where increased levels of growth hormone are desired, the problem has generally been approached by providing exogenous growth hormone or by administering a compound that stimulates the production or secretion of growth hormone. Typically, these compounds were peptidyl in nature and needed to be administered by injection. As an alternative approach, compounds termed secretagogues, have been developed that stimulate the production and/or release of endogenous hypothalamus growth hormone.

### Summary of the Invention

The present invention provides methods of administering a growth hormone secretagogue to a patient, the methods comprising the step of intermittently administering to a patient in need of growth hormone secretagogue administration a therapeutically effective amount of a growth hormone secretagogue.

In a preferred embodiment of the methods, the patient is a human.

In a preferred embodiment of the methods, the growth hormone secretagogue is administered every second day.

In another preferred embodiment of the methods, the growth hormone secretagogue is administered every third day.

In another preferred embodiment of the methods, the growth hormone secretagogue is administered three times a week.

In another preferred embodiment of the methods, the growth hormone secretagogue is administered four times a week.

In another preferred embodiment of the methods, the growth hormone secretagogue is administered every fourth day or every fifth day.

In another preferred embodiment of the methods, the growth hormone secretagogue is administered orally.

In another preferred embodiment of the methods, the growth hormone secretagogue is administered using an immediate release formulation.

In another preferred embodiment of the methods, the growth hormone secretagogue is administered using a controlled release formulation.

In a preferred embodiment of the methods wherein the growth hormone secretagogue is administered using a controlled release formulation, the controlled release formulation is a sustained release formulation, a delayed release formulation, or a combination thereof.

In another preferred embodiment of the methods, the growth hormone secretagogue is administered using a combination of an immediate release and a controlled release formulation.

In another preferred embodiment of the methods, the growth hormone secretagogue is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide or a pharmaceutically acceptable salt or prodrug thereof, or salt of the prodrug.

In another preferred embodiment of the methods, the growth hormone secretagogue is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate.

In another preferred embodiment of the methods, the growth hormone secretagogue is 2-amino-N-{1-(R)-(2,4-difluoro-benzyloxymethyl)-2-oxo-2-[3-oxo-3a-(R)-pyridin-2-ylmethyl-2-(2,2,2-trifluoro-ethyl)-2,3,3a,4,6,7-hexahydro-pyrazolo[4,3-c]pyridin-5-yl]-ethyl}-2-methyl-propionamide or a pharmaceutically acceptable salt or prodrug thereof, or salt of the prodrug.

In another preferred embodiment of the methods, the growth hormone secretagogue is the (L)-(+)-tartaric acid salt of 2-amino-N-{1-(R)-(2,4-difluoro-benzyloxymethyl)-2-oxo-2-[3-oxo-3a-(R)-pyridin-2-ylmethyl-2-(2,2,2-trifluoro-ethyl)-2,3,3a,4,6,7-hexahydro-pyrazolo[4,3-c]pyridin-5-yl]-ethyl}-2-methyl-propionamide.

In another preferred embodiment of the methods, the growth hormone secretagogue is administered using a tablet or capsule.

In another preferred embodiment of the methods, the growth hormone is administered once, twice or three times daily on the days of administration.

Also provided are methods of administering 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate to a patient in need thereof, the methods comprising the step of intermittently administering a therapeutically effective amount of 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate.

In a preferred embodiment of the methods of administering 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate to a patient in need thereof, the 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate is administered every second day.

In another preferred embodiment of the methods of administering 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate to a patient in need thereof, the 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate is administered every third day.

In another preferred embodiment of the methods of administering 2-amino-N-[2-(3a-(R)-benzyl-2-methyl-3-oxo-2,3,3a,4,6,7-hexahydro-pyrazolo[4,3-c]pyridin-5-yl)-1R-benzyloxymethyl-2-oxo-ethyl]-isobutyramide L-tartrate to a patient in need thereof, the 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate is administered orally in a tablet or capsule.

In a preferred embodiment of the methods of administering 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate to a patient in need thereof, the 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]- isobutyramide L-tartrate is administered in an amount in the range of about 1 mg to about 10 mg per day on the days of administration.

In a preferred embodiment of the methods of administering 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate to a patient in need thereof, the intermittent administration of 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate results in a peak plasma concentration in humans of growth hormone of about 5ng/ml or greater.

In a preferred embodiment of the methods of administering a growth hormone secretagogue to a patient, the patient is obese or has musculoskeletal frailty, osteoporosis, congestive heart failure or insulin resistance.

The present invention also provides a kit that comprises:
a. a composition comprising a growth hormone secretagogue;
b. a package for containing the composition; and
c. instructions for intermittently administering the composition comprising the growth hormone secretagogue.

In a preferred embodiment of the kit, the growth hormone secretagogue is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate.

In another preferred embodiment, the kit further comprises, in addition to a growth hormone secretagogue, an additional compound that can be used to treat obesity, musculoskeletal frailty, osteoporosis, congestive heart failure or insulin resistance.

In another preferred embodiment of the kit, the composition comprising a growth hormone secretagogue is a tablet or capsule.

### Detailed Description of the Invention

The present invention provides methods of administering a growth hormone secretagogue to a patient, the methods comprising the step of intermittently administering to a patient in need of growth hormone secretagogue administration a therapeutically effective amount of a growth hormone secretagogue. In a preferred embodiment of the invention, the intermittent administration is on non-consecutive days.

A growth hormone secretagogue, when administered to a patient, stimulates the production and/or secretion of growth hormone. In addition, growth hormone secretagogue administration typically increases the plasma concentration of insulin-like growth factor-1 (IGF-1).

Daily administration of a growth hormone secretagogue using a controlled release, an immediate release, or a combination of a controlled release and an immediate release formulation results in increased plasma concentrations of growth hormone; however, average peak plasma concentrations of growth hormone decline over time, usually to average peak plasma concentrations of less than 2 ng/ml in humans, despite the fact that IGF-1 plasma concentrations are increased with respect to placebo and do not appear to decline over time to the same extent as growth hormone. It is believed that obtaining average peak plasma concentrations of growth hormone of about 5 ng/ml or greater is therapeutically desirable.

It has unexpectedly and surprisingly been found that intermittent administration of a therapeutically effective amount of a growth hormone secretagogue in humans results in average peak plasma concentrations of growth hormone of 5 ng/ml or greater. In other words, it has been unexpectedly and surprisingly found that higher plasma concentrations of growth hormone can be obtained using intermittent administration instead of daily administration. Interestingly, with intermittent administration, plasma concentrations of IGF-1 are increased slightly when compared to placebo, but are not increased to the extent associated with daily administration. In addition to obtaining improved peak plasma concentrations of growth hormone over time while retaining the desired biological effects, intermittent administration ameliorates some of the unwanted effects seen with daily dosing. For example, glucose intolerance, edema, and hypertension are reduced or not present when a growth hormone secretagogue is administered intermittently when compared with daily dosing. Thus, intermittent dosing provides for improved plasma concentrations of growth hormone over time and results in lower occurrences of unwanted effects. The use of intermittent administration is preferred for growth hormone secretagogues that have a half-life of less than about twenty-four hours. More preferably, intermittent administration is useful for growth hormone secretagogues having a half-life of less than about twelve hours.

The term "intermittent" means that a growth hormone secretagogue or a combination of growth hormone secretagogues is not administered every day, as is typical of most medicines, but is administered every second day, every third day, every fourth day, twice a week, three times a week, four times a week, and so on. The term intermittent also includes administration of a growth hormone secretagogue on consecutive days followed by a period of days when no growth hormone is administered. For example, a growth hormone secretagogue can be administered on day one and two and then again on days five and six, and so on. Many variations of intermittent administration will be apparent to those skilled in the art; the present invention is intended to encompass such variations.

The intermittent administration of a growth hormone secretagogue in accordance with the present invention is exemplified below. To illustrate, a growth hormone secretagogue can be administered to a patient every third day. On the first day of therapy, a growth hormone secretagogue is administered. On the second and third day, no growth hormone secretagogue is administered. On the fourth day, growth hormone secretagogue is administered again. Similarly, growth hormone secretagogue can be administered every second day. On the first day growth hormone secretagogue is administered. On the second day no growth hormone secretagogue is administered, and on the third day growth hormone secretagogue is administered again, and so on. A preferred embodiment of the present invention is administration of a growth hormone secretagogue three times a week, preferably using an immediate release formulation.

The term "patient" means animals, such as dogs, cats, cows, horses, sheep, and humans. Preferred patients are mammals, including both males and females with humans being even more preferred.

The term "pharmaceutically acceptable" means that a substance or mixture of substances must be compatible with the other ingredients of a formulation, and not deleterious to a patient.

The terms "treating", "treat" or "treatment" include preventive (e.g., prophylactic) and palliative treatment.

The term "therapeutically effective amount" means an amount of a growth hormone secretagogue that ameliorates, attenuates, or eliminates one or more diseases or conditions associated with growth hormone secretion.

The term "immediate release" means that most of the active compound or compounds become available to the patient for absorption and distribution relatively soon after administration. In a typical immediate release formulation using an orally ingested tablet or capsule, the active compound or compounds are released in the stomach.

The term "controlled release" means that the active compound or compounds become available to a patient for absorption and distribution at a particular time after administration, at a particular place in the gastrointestinal tract, or over a period of time or combinations thereof. The term controlled release includes sustained release, delayed release and combinations thereof.

The term "delayed release" means that the active compound or compounds are not immediately released for absorption and distribution, for example in the stomach, but are released at some time period after administration or at a particular place in the gastrointestinal tract. To illustrate, a formulation can be designed so that release of the active compound or compounds occurs in the small intestine. Alternatively, a formulation can be designed so that release of the active compound or compounds occurs about one hour after administration.

The term "sustained release" means that active compound or compounds are released over a period of time. Preferably, the amount of compound released over a given time period is relatively constant. To illustrate, a formulation containing about 60 mg of an active compound can be designed to deliver about 10 mg of active compound every hour for about 6 hours.

It is also recognized that a formulation may have the properties of delayed release and sustained release. For example, a formulation can be designed to release active compound in the small intestine rather than the stomach and then release the active compound in the small intestine over a period of time. In addition, a formulation can include both an immediate release portion and a controlled release portion. For example, a tablet can be designed to release a certain quantity of an active compound immediately in the stomach upon oral ingestion and then deliver a quantity of the active compound over a time period in the intestine. The design, use and manufacture of immediate release and controlled release formulations, including sustained and/or delayed release formulations or combinations thereof, are well known to those in the art.

The terms "active compound" or "active agent" means a compound that exerts a pharmacological effect on a patient. These terms are intended to include salts and prodrugs of the compound and salts of the prodrugs.

A preferred patient in need of growth hormone secretagogue administration is a patient having frailty, osteoporosis, congestive heart failure, or insulin resistance, or a patient who is obese.

The administration of a growth hormone secretagogue to a patient also accelerates bone fracture repair, attenuates protein catabolic response after surgery, reduces cachexia and protein loss due to a chronic illness such as AIDS and cancer, accelerates wound healing, and accelerates the recovery of burn victims or persons having undergone major surgery. The administration of a growth hormone secretagogue is also known to enhance the quality of sleep, which is disclosed in WO 97/24369. A growth hormone secretagogue can be administered to a patient having one or more of the conditions or symptoms recited above. Uses of growth hormone are summarized in PCT publication WO 97/24369.

The growth hormone secretagogue administration methods of the present invention can be used to treat any disease or condition that can be treated by the administration of a growth hormone secretagogue. It is also noted that a growth hormone secretagogue can be administered to a non-human animal, and such administration is useful to treat the symptoms of growth hormone deficiency, stimulate growth and/or enhance the feeding efficiency of animals raised for meat production, improve carcass quality, increase milk production, improve bone and wound healing, and improve the animal's protein to fat ratio. Thus, in accordance with the present invention, a growth hormone secretagogue can be intermittently administered to a non-human animal need of growth hormone administration.

A growth hormone secretagogue is a compound that, when administered to a patient, increases the secretion of growth hormone when compared with baseline plasma concentrations of growth hormone. Thus, to identify a growth hormone secretagogue, one need simply measure the baseline plasma concentrations of growth hormone over a time period, typically one day, and compare the plasma concentrations of growth hormone after administration of a growth hormone secretagogue with the baseline concentration over the time period. Various examples of growth hormone secretagogues are disclosed herein. It is contemplated that any growth hormone secretagogue can be used in the present administration methods. Preferred growth hormone secretagogues that can be used in the present invention have an *in vivo* half-life in humans of less than about twenty-four hours and more preferably less then about 12 hours.

The following patents and applications disclose growth hormone secretagogues that can be used alone or in combination with other growth hormone secretagogues or other therapeutically active compounds in the methods of the present invention: WO 98/46569, WO 98/51687, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 97/00894, WO 97/07117, WO 97/06803, WO 97/11697, WO 97/15573, WO 97/22367, WO 97/23508, WO 97/22620, WO 97/22004, WO 97/21730, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, WO 96/38471, WO 96/35713, U.S. 5,919,777, and U.S. 5,830,433.

In addition, the following growth hormone secretagogues are contemplated for use in the present invention: MK-0677 (Merck); NM703 (Novo Nordisk); L-162752 and L-163022 (Merck); hexarelin (Pharmacia & Upjohn); GPA-748, KP102, and GHRP-2 (American Home Products); ipamorelin (Novo Nordisk); LY444711 (Eli Lilly); Geref (Ares/Serono); GHRH (1-44) [BioNebraska]; Somatorelin (GRF 1-44) [Fujisawa/ICN]; and ThGRF (Theratechnologies).

Particularly preferred compounds for use in the present invention are those disclosed in WO 97/24369 and WO 98/58947, including 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide; 2-amino-N-{1-(R))-(2,4-difluoro-benzyloxymethyl)-2-oxo-2-[3-oxo-3a-(R)-pyridin-5-ylmethyl-2-(2,2,2-trifluoro-ethyl)-2,3,3a,4,6,7-hexahydro-pyrazolo[4,3-c]pyridin-5-yl]-ethyl}-2-methyl-propionamide; 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate, and the (L)-(+)-tartaric acid salt of 2-amino-N-{1-(R)-(2,4-difluoro-benzyloxymethyl)-2-oxo-2-[3-oxo-3a-(R)-pyridin-2-ylmethyl-2-(2,2,2-trifluoro-ethyl)-2,3,3a,4,6,7-hexahydro-pyrazolo[4,3-c]pyridin-5-yl]-ethyl}-2-methyl-propionamide.

A growth hormone secretagogue can be administered alone or as part of a pharmaceutically acceptable composition. As used herein, the term composition is synonymous with the term formulation. The compositions can be administered all at once, as for example, by a bolus injection, or multiple times, such as by a series of tablets. In addition, a single growth hormone secretagogue can be administered, or a growth hormone secretagogue can be administered in combination with other growth hormone secretagogues, or with other pharmaceutically active compounds.

The other pharmaceutically active compounds can be intended to treat the same disease or condition as a growth hormone secretagogue or a different disease or condition. For example, a growth hormone secretagogue can be administered in combination with other agents used to treat frailty, osteoporosis, congestive heart failure, wound healing, insulin resistance, and the like.

If the patient is to receive or is receiving multiple pharmaceutically active compounds, the compounds can be administered simultaneously or sequentially in any order. For example, in the case of tablets, the active compounds may be found in one tablet or in separate tablets, which can be administered at once or sequentially in any order. In addition, it should be recognized that the compositions can be different forms. For example, one or more compounds can be delivered via a tablet, while another is administered via injection or orally as a syrup. In accordance with the present invention, the growth hormone secretagogue should be intermittently administered. It is noted that any other active compound that is not a growth hormone secretagogue can be intermittently administered, usually along with the growth hormone secretagogue, or administered daily. To illustrate, a growth hormone secretagogue can be administered in combination with an additional compound that is useful to treat osteoporosis. The growth hormone secretagogue is intermittently administered, and the additional compound can be administered daily. In other words, the administration schedule of any additional compound can be independent of the administration schedule of the growth hormone secretagogue.

A growth hormone secretagogue and other pharmaceutically active compounds used in combination with a growth hormone secretagogue, if desired, can be administered to a patient either orally, rectally, parenterally, (for example, intravenously, intramuscularly, or subcutaneously) intracistemally, intravaginally, intraperitoneally, intravesically, locally (for example, powders, ointments or drops), or as a buccal or nasal spray. A preferred mode of administration is orally via a tablet or capsule.

Compositions suitable for parenteral injection may comprise physiologically acceptable sterile aqueous or non-aqueous solutions, dispersions, suspensions, or emulsions, and sterile powders for reconstitution into sterile injectable solutions or dispersions. Examples of suitable aqueous and non-aqueous carriers, diluents, solvents, or vehicles include water, ethanol, polyols (propylene glycol, polyethylene glycol, glycerol, and the like), suitable mixtures thereof, triglycerides, including vegetable oils such as olive oil, and injectable organic esters such as ethyl oleate. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Prevention of microorganism contamination of the compositions can be ensured by various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents delaying absorption, for example, aluminum monostearate and gelatin.

Solid dosage forms for oral administration include capsules, tablets, powders, and granules. In such solid dosage forms, the active compound is admixed with at least one inert customary excipient (or carrier) such as sodium citrate or dicalcium phosphate or (a) fillers or extenders, as for example, starches, lactose, sucrose, mannitol, or silicic acid; (b) binders, as for example, carboxymethylcellulose, alginates, gelatin, polyvinylpyrrolidone, sucrose, or acacia; (c) humectants, as for example, glycerol; (d) disintegrating agents, as for example, agar-agar, calcium carbonate, potato or tapioca starch, alginic acid, certain complex silicates, or sodium carbonate; (e) solution retarders, as for example, paraffin; (f) absorption accelerators, as for example, quaternary ammonium compounds; (g) wetting agents, as for example, cetyl alcohol or glycerol monostearate; (h) adsorbents, as for example, kaolin or bentonite; and/or (i) lubricants, as for example, talc, calcium stearate, magnesium stearate, solid polyethylene glycols, sodium lauryl sulfate, or mixtures thereof. In the case of capsules and tablets, the dosage forms may also comprise buffering agents.

Solid compositions of a similar type may also be used as fillers in soft or hard filled gelatin capsules using such excipients as lactose or milk sugar, as well as high molecular weight polyethylene glycols, or the like.

Solid dosage forms such as tablets, dragees, capsules, and granules can be prepared with coatings and shells, such as enteric coatings and others well known in the art. They may also contain opacifying agents, and can also be of such composition that they release the active compound or compounds in a delayed and/or sustained manner as described above. Examples of embedding compositions that can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, Miglyol® , glycerol, tetrahydrofurfuryl alcohol, polyethylene glycols and fatty acid esters of sorbitan, or mixtures of these substances, or the like.

Besides such inert diluents, the composition can also include adjuvants, such as wetting agents, emulsifying and suspending agents, sweetening, flavoring, and perfuming agents.

Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, or tragacanth, or mixtures of these substances, or the like.

Dosage forms for topical administration of a compound of the present invention include ointments, powders, sprays and inhalants. The active compound or compounds are admixed under sterile conditions with a physiologically acceptable carrier, and any preservatives, buffers, or propellants that may be required.

The growth hormone secretagogues can be administered to a patient at dosage levels in the range of about 0.01 to about 7,000 mg per day. For a normal adult human having a body weight of about 70 kg, a dosage in the range of about 0.01 to about 100 mg per kilogram body weight is typically sufficient. The specific dosage and dosage range that can be used depends on a number of factors, including the requirements of the patient, the severity of the condition or disease being treated, and the pharmacological activity of the growth hormone secretagogues being administered. The determination of dosage ranges and optimal dosages for a particular patient is well within the ordinary skill in the art. A preferred dosage range for a growth hormone secretagogue in humans is about 0.1 to about 700 mg per day of administration. A more preferred dosage range is about 0.5 to about 25 mg per day of administration. An even more preferred dosage range is about 1 to about 10 mg per day of administration.

The following paragraphs describe exemplary formulations, dosages etc. useful for non-human animals. The administration of a growth hormone secretagogue can be effected orally or non-orally, for example by injection. An amount of a compound, also referred to below as "agent," is administered such that an effective dose is received, generally a dose which, when administered orally to an animal is usually between 0.01 and 100 mg/kg/day of body weight, preferably between 0.1 and 50 mg/kg/day of body weight. Conveniently, the medication can be carried in the drinking water so that a therapeutic dosage of the agent is ingested with the daily water supply. The agent can be directly metered into drinking water, preferably in the form of a liquid, water-soluble concentrate (such as an aqueous solution of a water soluble salt). Conveniently, the agent can also be added directly to the feed, as such, or in the form of an animal feed supplement, also referred to as a premix or concentrate. A premix or concentrate of a therapeutic agent in a carrier is more commonly employed for the inclusion of the agent in the feed. Suitable carriers are liquid or solid, as desired, such as water, various meals such as alfalfa meal, soybean meal, cottonseed oil meal, linseed oil meal, corncob meal and corn meal, molasses, urea, bone meal, or mineral mixes such as are commonly employed in poultry feeds. A particularly effective carrier is the respective animal feed itself; that is, a small portion of such feed. The carrier facilitates uniform distribution of the agent in the finished feed with which the premix is blended. It is important that the agent be thoroughly blended into the premix and, subsequently, the feed. In this respect, the agent may be dispersed or dissolved in a suitable oily vehicle such as soybean oil, corn oil, cottonseed oil, and the like, or in a volatile organic solvent and then blended with the carrier. It will be appreciated that the proportions of the agent in the concentrate are capable of wide variation since the amount of agent in the finished feed may be adjusted by blending the appropriate proportion of premix with the feed to obtain a desired level of agent.

High potency concentrates may be blended by the feed manufacturer with a proteinaceous carrier such as soybean oil meal and other meals, as described above, to produce concentrated supplements, which are suitable for direct feeding to animals. In such instances, the animals are permitted to consume the usual diet. Alternatively, such concentrated supplements may be added directly to the feed to produce a nutritionally balanced, finished feed containing a therapeutically effective level of a compound according to the invention. The mixtures are thoroughly blended by standard procedures, such as in a twin shell blender, to ensure homogeneity.

If the supplement is used as a top dressing for the feed, it likewise helps to ensure uniformity of distribution of the active material across the top of the dressed feed.

Preferred medicated swine, cattle, sheep and goat feeds generally contain from about 1 to about 400 grams of active ingredient per ton of feed, the optimum amount for these animals usually being about 50 to about 300 grams per ton of feed.

Preferred poultry and domestic pet feeds usually contain about 1 to about 400 grams and preferably about 10 to about 400 grams of active ingredient per ton of feed.

For parenteral administration in animals, a growth hormone secretagogue may be prepared in the form of a paste or a pellet and administered as an implant, usually under the skin of the head or ear of the animal.

In general, parenteral administration involves injection of a sufficient amount of a growth hormone secretagogue to provide the animal with about 0.01 to about 100 mg/kg/day of body weight of the active ingredient. The preferred dosage for poultry, swine, cattle, sheep, goats and domestic pets is in the range of from about 0.1 to about 50 mg/kg/day.

Paste formulations can be prepared by dispersing the active compound in a pharmaceutically acceptable oil such as peanut oil, sesame oil, corn oil, or the like.

Pellets containing an effective amount of a growth hormone secretagogue can be prepared by admixing a growth hormone secretagogue with a diluent such as carbowax, carnauba wax, and the like, and a lubricant, such as magnesium or calcium stearate, can be added to improve the pelleting process.

Preferred growth hormone secretagogues for administration to non-human animals include those disclosed in WO 98/58947.

A growth hormone secretagogue can be administered to a patient as a pharmaceutically acceptable salt or as a prodrug. The terms pharmaceutically acceptable salt or prodrug mean the salts or prodrugs of a growth hormone secretagogue that are, within the scope of sound medical judgment, suitable for use with patients without undue toxicity, irritation, allergic response, and the like, commensurate with a reasonable benefit/risk ratio, and effective for their intended use, as well as the zwitterionic forms, where possible.

The term "salts" refers to inorganic and organic salts of a growth hormone secretagogue. Such salts can be prepared *in situ* during the final isolation and purification of a compound, or by separately reacting a purified compound with a suitable organic or inorganic acid or base, as required, and isolating the salt thus formed. Representative salts include the hydrobromide, hydrochloride, sulfate, bisulfate, nitrate, acetate, oxalate, palmitate, stearate, laurate, borate, benzoate, lactate, phosphate, tosylate, besylate, esylate, citrate, maleate, fumarate, succinate, tartrate, naphthylate, mesylate, glucoheptonate, lactobionate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S.M. Berge, *et al.,* "Pharmaceutical Salts," *J Pharm Sci,* **66**:1-19 (1977).

The term "prodrug" means a compound that is transformed *in vivo* to yield a growth hormone secretagogue. The transformation may occur by various mechanisms, such as through hydrolysis in blood. A discussion of the use of prodrugs is provided by T. Higuchi and W. Stella, "Pro-active compounds as Novel Delivery Systems," Vol. 14 of the *A.C.S. Symposium Series,* and in *Bioreversible Carriers in Active compound Design,* ed. Edward B. Roche, American Pharmaceutical Association and Pergamon Press, 1987.

For example, if a growth hormone secretagogue contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a growth hormone secretagogue comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a growth hormone secretagogue comprises an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, or benzyl, or R-carbonyl is a natural α-aminoacyl or natural α-aminoacyl-natural α-aminoacyl, -C(OH)C(O)OY wherein Y is H, (C₁-C₆)alkyl or benzyl, -C(OY₀)Y₁ wherein Y₀ is (C₁-C₄) alkyl and Y₁ is ((C₁-C₆)alkyl, carboxy(C₁-C₆)alkyl, amino(C₁-C₄)alkyl or mono-N- or di-N,N-(C₁-C₆)alkylaminoalkyl, -C(Y₂)Y₃ wherein Y₂ is H or methyl and Y₃ is mono-N- or di-N,N-(C₁-C₆)alkylamino, morpholino, piperidin-1-yl or pyrrolidin-1-yl.

A growth hormone secretagogue may contain asymmetric or chiral centers, and therefore, exist in different stereoisomeric forms. It is contemplated that all stereoisomeric forms of a growth hormone secretagogue as well as mixtures thereof, including racemic mixtures, form part of the present invention. In addition, the present invention contemplates all geometric and positional isomers. For example, if a growth hormone secretagogue contains a double bond, both the cis and trans forms, as well as mixtures, are contemplated.

Mixtures of isomers, including stereoisomers can be separated into their individual components on the basis of their physical chemical differences by methods well know to those skilled in the art, such as by chromatography and/or fractional crystallization. Enantiomers can be separated by converting the enantiomeric mixture into a diasteromeric mixture by reaction with an appropriate optically active compound (e.g., alcohol), separating the diastereomers and converting (e.g., hydrolyzing) the individual diastereomers to the corresponding pure enantiomers. Also, some of the compounds of this invention may be atropisomers (e.g., substituted biaryls) and are considered as part of this invention.

A growth hormone secretagogue may exist in unsolvated as well as solvated forms with pharmaceutically acceptable solvents such as water, ethanol, and the like. The present invention contemplates and encompasses both the solvated and unsolvated forms.

It is also possible that a growth hormone secretagogue may exist in different tautomeric forms. All tautomers of a growth hormone secretagogue are contemplated. For example, all of the tautomeric forms of the imidazole moiety are included in this invention. Also, for example, all keto-enol and/or imine-enamine forms of a growth hormone secretagogue are included in this invention. Those skilled in the art will recognize that any compound names contained herein may be based on a particular tautomer of a compound. While the name for only a particular tautomer may be used, it is intended that all tautomers are encompassed by the name of the particular tautomer and all tautomers are considered part of the present invention.

The present invention also includes isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶CI, respectively. Growth hormone secretagogues that contain the aforementioned isotopes and/or other isotopes of other atoms are within the scope of this invention. Certain isotopically-labelled growth hormone secretagogues, for example those into which radioactive isotopes such as ³H and ¹⁴C are incorporated, are useful in active compound and/or substrate tissue distribution assays. Tritiated, i.e., ³H, and carbon-14, i.e., ¹⁴C, isotopes are particularly preferred for their ease of preparation and detection. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

A growth hormone secretagogue can be administered in combination with one or more other growth hormone secretagogues or other pharmacologically active compounds. Preferred combinations include a growth hormone secretagogue and a compound used to treat the same diseases or conditions as growth hormone secretagogues. For example, a growth hormone secretagogue can be used to treat osteoporosis. Other compounds that are known to be useful to treat osteoporosis and that can be used in combination with a growth hormone secretagogue include estrogen agonists / antagonists [also called selective estrogen receptor modulators (SERMs)], such as tamoxifen, droloxifene, lasofoxifene (U.S. 5,552,412), raloxifene, idoxifene, and bisphosphonates such as alendronate, tiludronate, dimethyl APD, risedronate, etidronate, YM-175, clodronate, pamidronate, and BM-210995 (ibandronate). Another class of compounds that can be used to treat osteoporosis and that can be used in combination with a growth hormone secretagogue are prostaglandin E2 agonists. Examples of prostaglandin E2 agonists that can be used in combination with a growth hormone secretagogue include those disclosed in WO 99/19300 and WO 98/28264. Other compounds that are used to treat osteoporosis that can be used in combination with a growth hormone secretagogue include Premarin® , progesterone, Evista® , calcitonin, and estrogen.

The terms estrogen agonist / antagonsit refer to compounds that bind with the estrogen receptor. In particular, estrogen agonists are compounds capable of binding to the estrogen receptor and mimicking the effects of estrogen. Estrogen antagonists are compounds that bind to the estrogen receptor and block the action of estrogen. Exemplary estrogen agonists / antagonists include droloxifene and the other compounds disclosed in U.S. patent no. 5,047,431, tamoxifen and the other compounds disclosed in U.S. patent nos. 4,536,516 and 4,623,660, and raloxifene and the other compounds disclosed in U.S. patent no. 4,839,155. In addition, the compounds disclosed in U.S. patent no. 5,552,412, and 4,133,814, which are estrogen agonists / antagonists, can be used in the present invention.

Preferred estrogen agonists / antagonists that can be used in the present invention include those disclosed in U.S. patent no. 5,552,412 such as:
*cis*-6-(4-fluoro-phenyl)-5-[4-(2-piperidin-1-yl-ethoxy)-phenyl]-5,6,7,8-tetrahydro-naphthalene-2-ol;
(-)-*cis*-6-phenyl-5-[4-(2-pyrrolidin-1-yl-ethoxy)-phenyl]-5,6,7,8-tetrahydro-naphthalene-2-ol;
*cis*-6-phenyl-5-[4-(2-pyrrolidin-1-yl-ethoxy)-phenyl]-5,6,7,8-tetrahydro-naphthalene-2-ol;
*cis*-1-[6'-pyrrolodinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
*cis*-6-(4-hydroxyphenyl)-5-[4-(2-piperidin-1-yl-ethoxy)-phenyl]-5,6,7,8-tetrahydro-naphthalene-2-ol; and
1-(4'-pyrrolidinoethoxyphenyl)-2-phenyl-6-hydroxy-1,2,3,4-tetrahydroisoquinoline.

A growth hormone secretagogue can also be used in combination with other growth promoting and anabolic agents such as thyrotropin-releasing hormone (TRH), parathyroid hormone (PTH), diethylstilbestrol, β-agonists, theophylline, anabolic steroids, enkephalins, E series prostaglandins, and the compounds disclosed in U.S. patent numbers 3,239,345; 4,036,979; and 4,411,890.

Growth hormone secretagogues that can be used alone in the present invention or used in combination with other growth hormone secretagogues include the growth hormone releasing peptides GHRP-2, GHRP-6 and GHRP-1. Growth hormone releasing hormone and growth hormone and their analogs can also be used in the present invention. Additional compounds that can be used in the present invention include hexarelin, somatomedins such as IGF-1 and IGF-2, or adrenergic agonists such as clonidine (U.S. 3,202,660), xylazine (U.S. 3,235,550), detomidine and medetomidine (U.S. 4,544,664), and serotonin 5HTID agonists such as sumitripan or agents that inhibit somatostatin or its release such as physostigmine and pyridostigmine.

A growth hormone secretagogue can also be used in combination with one or more compounds that are useful to treat obesity. Examples of classes of compounds that can be used to treat obesity include the active compound(s) in appetite suppressants such as Adipex® , Bontril® , Desoxyn Gradumet® , Fastin® , lonamin® , and Meridia® , and lipase inhibitors such as Xenical® .

Additional anti-obesity agents that can be used in combination with a growth hormone secreatagogue include a β₃-adrenergic receptor agonist, a cholecystokinin-A agonist, a monoamine reuptake inhibitor, a sympathomimetic agent, a serotoninergic agent, a dopamine agonist, a melanocyte-stimulating hormone receptor agonist or mimetic, a melanocyte-stimulating hormone receptor analog, a cannabinoid receptor antagonist, a melanin concentrating hormone antagonist, leptin, a leptin analog, a leptin receptor agonist, a galanin antagonist, a bombesin agonist, a neuropeptide-Y antagonist (including NPY-1 and NPY-5), a thyromimetic agent, dehydroepiandrosterone or an analog thereof, a glucocorticoid receptor agonist or antagonist, an orexin receptor antagonist, a urocortin binding protein antagonist, a glucagon-like peptide-1 receptor agonist, and a ciliary neurotrophic factor.

Especially preferred anti-obesity agents that can be used in combination with a growth hormone secretagogue include compounds selected from the group consisting of sibutramine, fenfluramine, dexfenfluramine, bromocriptine, phentermine, ephedrine, leptin, phenylpropanolamine pseudoephedrine, {4-[2-(2-[6-aminopyridin-3-yl]-2(R)-hydroxyethylamino)ethoxy]phenyl}acetic acid, {4-[2-(2-[6-aminopyridin-3-yl]-2(R)-hydroxyethylamino)ethoxy]phenyl}benzoic acid, {4-[2-(2-[6-aminopyridin-3-yl]-2(R)-hydroxyethylamino)ethoxy]phenyl}propionic acid, and {4-[2-(2-[6-aminopyridin-3-yl]-2(R)-hydroxyethylamino)ethoxy]phenoxy}acetic acid.

Examples of thyromimetics that can be used in combination with a growth hormone secretagogue include those disclosed in U.S. provisional patent application numbers 60/178,968 and 60/177,987.

Examples of glucocorticoid receptor ligands that can be used in combination with a growth hormone secretagogue include those disclosed in U.S. provisional patent application number 60/132,130.

Examples of neuropeptide-Y antagonists that can be used in combination with a growth hormone secretagogue include those disclosed in WO 98/23603, U.S. 5,900,415, U.S. 5,914,329, and U.S. provisional patent application number 60/132,029 (NPY-5).

Examples of β₃-adrenergic receptor agonists that can be used in combination with a growth hormone secretagogue include those disclosed in WO 96/35671.

A growth hormone secretagogue can also be used in combination with a compound that is useful to treat congestive heart failure. Examples of classes of compounds that are useful to treat heart failure include diuretics, angiotensin converting enzyme (ACE) inhibitors, angiotensin II receptor blockers, digitalis, β-blockers, and corticotropin releasing factor (CRF) antagonists. Examples of CRF antagonists that can be used in combination with a growth hormone secretagogue are disclosed in WO 95/33750. Preferred compounds disclosed in WO 95/33750 include:
4-(1-ethyl-propoxy)-2,5-dimethyl-6-(2,4,6-trimethyl-benzyl)-pyrimidine;
2-(4-bromo-2,6-dimethyl-phenoxy)-4-(1-ethyl-propoxy)-3,6-dimethyl-pyridine;
2-(4-ethyl-2,6-dimethyl-phenoxy)-4-(1-ethyl-propoxy)-3,6-dimethyl-pyridine;
3-ethyl-4-(1-ethyl-propoxy)-6-methyl-2-(2,4,6-trimethyl-phenoxy)-pyridine;
2-(2,6-dimethyl-4-propyl-phenoxy)-4-(1-ethyl-propoxy)-3,6-dimethyl-pyridine;
4-(1-ethyl-propoxy)-2-(4-methoxy-2,6-dimethyl-phenoxy)-3,6-dimethyl-pyridine;
2-(4-ethoxy-2,6-dimethyl-phenoxy)-4-(1-ethyl-propoxy)-3,6-dimethyl-pyridine;
2-(4-chloro-2,6-dimethyl-phenoxy)-4-(1-ethyl-propoxy)-3,6-dimethyl-pyridine;
4-(1-methoxymethyl-propoxy)-3,6-dimethyl-2-(2,4,6-trimethyl-phenoxy)-pyridine;
[3,6-dimethyl-2-(2,4,6-trimethyl-phenoxy)-pyridin-4-yl]-diethyl-amine;
[3,6-dimethyl-2-(2,4,6-trimethyl-phenoxy)-pyridin-4-yl]-ethyl-propyl-amine;
[2,5-dimethyl-6-(2,4,6-trimethyl-phenoxy)-pyrimidin-4-yl](1-ethyl-propyl)-amine;
butyl-[3,6-dimethyl-2-(2,4,6-trimethyl-phenoxy)-pyridin-4-yl]-ethyl-amine;
4-(1-ethyl-propoxy)-3,6-dimethyl-2-(2,4,6-trimethyl-phenylsulfanyl)-pyridine;
butyl-[2-(4-chloro-2,6-dimethyl-phenoxy)-3,6-dimethyl-pyridin-4-yl]-ethyl-amine;
4-(1-ethyl-propylamino)-6-methyl-2-(2,4,6-trimethyl-phenoxy)-nicotinic acid methyl ester;
[3,6-dimethyl-[2-(2,4,6-trimethyl-phenylsulfanyl)-pyridin-4-yl]-ethyl-propyl-amine;
4-(1-ethyl-propylamino)-6-methyl-2-(2,4,6-trimethyl-phenoxy)-pyridine-3-yl]-methanol;
[2-(4-chloro-2,6-dimethyl-phenoxy)-3,6-dimethyl-pyridin-4-yl]-ethyl-propyl-amine;
1-(ethyl-propyl)-[6-methyl-3-nitro-2-(2,4,6-trimethyl-phenoxy)pyridin-4-yl]-amine;
N4-(1-ethyl-propyl)-6-methyl-3-nitro-N2-(2,4,6-trimethyl-phenyl)-pyridine-2,4-diamine;
N4-(1-ethyl-propyl)-6-methyl-2-(2,4,6-trimethyl-phenoxy)-pyridine-3,4-diamine;
3,6-dimethyl-2-(2,4,6-trimethyl-phenoxy)-pyridin-4-yl]-ethyl-(2,2,2-trifluoro-ethyl)-amine;
N4-(1-ethyl-propyl)-6-methyl-N2-(2,4,6-trimethyl-phenyl)pyridine-2,3,4-triamine;
[3-chloromethyl-6-methyl-2-(2,4,6-trimethyl-phenoxy)pyridin-4-yl]-(1-ethyl-propyl)-amine;
[3,6-dimethyl-2-(2,4,6-trimethyl-phenoxy)-pyridin-4-yl]-(1-ethyl-propyl)-amine;
(1-ethyl-propyl)-[2-methyl-5-nitro-6-(2,4,6-trimethyl-pyridin-3-yloxy)-pyrimidin-4-yl]-amine;
(1-ethyl-propyl)-[3-methoxymethyl-6-methyl-2-(2,4,6-trimethyl-phenoxy)-pyridin-4-yl]-amine;
(N-(1-ethyl-propyl)-2-methyl-5-nitro-N'-(2,4,6-trimethyl-pyridin-3-yl)-pyrimidine-4,6-diamine;
[2-(4-chloro-2,6-dimethyl-phenoxy)-3,6-dimethyl-pyridin-4-yl]-diethyl-amine;
4-(1-ethyl-propoxy)-3,6-dimethyl-2-(2,4,6-trimethylphenoxy)-pyridine;
butyl-[2,5-dimethyl-7-(2,4,6-trimethylphenyl)-6,7-dihydro-5H-pyrrolo[2,3-d]pyrimidin-4-yl]-ethyl-amine;
4-(butyl-ethylamino)-2,5-dimethyl-7-(2,4,6-trimethylphenyl-5,7-dihydro-pyrrolo [2,3-d]pyrimidin-6-one;
4-(1-ethylpropoxy)-2,5-dimethyl-6-(2,4,6-trimethylphenoxy)-pyrimidine;
N-butyl-N-ethyl-2,5-dimethyl-N'-(2,4,6-trimethylphenyl)-pyrimidine-4,6-diamine;
(1-ethyl-propyl)-[5-methyl-3-(2,4,6-trimethyl-phenyl)-3H-imidazo[4,5-b]pyridin-7-yl]-amine;
[2,5-dimethyl-3-(2,4,6-trimethyl-phenyl)-3H-imidazo[4,5-b]pyridin-7-yl]-(1-ethylpropyl)-amine;
N4-(1-ethyl-propyl)-6,N3-dimethyl-2-(2,4,6-trimethyl-phenoxy)-pyridine-3,4-diamine;
N4-(1-ethyl-propyl)-6,N3, N3-trimethyl-2-(2,4,6-trimethyl-phenoxy)-pyridine-3,4-diamine;
6-(1-ethyl-propoxy)-2-methyl-N4-(2,4,6-trimethyl-phenyl)-pyrimidine-4,5-diamine;
[4-(1-ethyl-propoxy)-3,6-dimethyl-pyridin-2-yl]-(2,4,6-trimethylphenyl)-amine; and
6-(ethyl-propyl-amino)-2,7-dimethyl-9-(2,4,6-trimethylphenyl)-7,9-dihydro-purin-8-one.

A growth hormone secretagogue can also be used in combination with a compound useful to treat insulin resistance. Representative agents that can be used include insulin and insulin analogs (e.g., LysPro insulin); GLP-1 (7-37) (insulinotropin) and GLP-1 (7-36)-NH₂; biguanides: metformin, phenformin, buformin; α2-antagonists and imidazolines: midaglizole, isaglidole, deriglidole, idazoxan, efaroxan, fluparoxan; sulfonylureas and analogs: chlorpropamide, glibenclamide, tolbutamide, tolazamide, acetohexamide, glypizide, glimepiride, repaglinide, meglitinide; other insulin secretagogues: linogliride, A-4166; glitazones: ciglitazone, pioglitazone, englitazone, troglitazone, darglitazone, rosiglitazone; PPAR-gamma agonists; fatty acid oxidation inhibitors: clomoxir, etomoxir; α-glucosidase inhibitors: acarbose, miglitol, emiglitate, voglibose, MDL-25,637, camiglibose, MDL-73,945; β-agonists: BRL 35135, BRL 37344, Ro 16-8714, ICI D7114, CL 316,243; phosphodiesterase inhibitors: L-386,398; lipid-lowering agents: benfluorex; antiobesity agents: fenfluramine; vanadate and vanadium complexes (e.g., Naglivan® ) and peroxovanadium complexes; amylin antagonists; glucagon antagonists; gluconeogenesis inhibitors; somatostatin analogs and antagonists; antilipolytic agents: nicotinic acid, acipimox, WAG 994. Also contemplated for use in combination with a growth hormone secretagogue are pramlintide acetate (Symlin™), AC2993, and nateglinide.

Since one aspect of the present invention contemplates the treatment of the disclosed diseases/conditions with a combination of pharmaceutically active agents that may be administered separately in any order, the invention further relates to combining separate pharmaceutical compositions in kit form. The kit comprises two separate pharmaceutical compositions: one composition containing a growth hormone secretagogue, and a second composition containing a second pharmaceutical compound. The kit comprises a container for containing the separate compositions such as a divided bottle or a divided foil packet. Additional examples of containers include syringes, boxes, bags, and the like. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil, which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen that the tablets or capsules so specified should be ingested. Another example of such a memory aid is a calendar printed on the card, e.g., as follows "First Week, Monday, Tuesday, ...etc.... Second Week, Monday, Tuesday," etc. Other variations of memory aids will be readily apparent. A "daily dose" can be a single tablet or capsule or several pills or capsules to be taken on a given day. Also, a daily dose of a growth hormone secretagogue can consist of one tablet or capsule, while a daily dose of the second compound can consist of several tablets or capsules and vice versa. The memory aid should reflect this and aid in correct administration of the active agents. It is also envisioned that even if a growth hormone secretagogue is administered alone (i.e., without additional active compounds), a kit may be provided to help a patient remember when to administer the growth hormone secretagogue, since administration is intermittent and help remembering when to take a dose may be useful. Thus, a kit may comprise a package containing dosages of a growth hormone secretagogue along with a means for helping the patient remember when to take the dose. For example, a dispenser designed to dispense the doses one at a time in the order of their intended use can be provided. Preferably, the dispenser is equipped with a memory-aid, so as to further facilitate compliance with the regimen. An example of such a memory-aid is a mechanical counter that indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

A growth hormone secretagogue or a combination of growth hormone secretagogues can be administered in accordance with the present invention using a sustained release formulation. For purposes of discussion, not limitation, the many embodiments hereunder can be grouped into classes according to design and principle of operation.

The first class of sustained release dosage forms described below is matrix systems, which include but are not limited to 1) non-eroding matrices, tablets, multiparticulates, and hydrogel-based systems; 2) hydrophilic eroding, dispersible or dissolvable matrix systems, tablets and multiparticulates; and 3) coated matrix systems. The second class comprises reservoir systems where release of the active compound is modulated by a membrane, such as capsules, and coated tablets or multiparticulates. The third class comprises osmotic-based systems such as 1) coated bilayer tablets; 2) coated homogeneous tablet cores; 3) coated multiparticulates; and 4) osmotic capsules. The fourth class comprises swellable systems where active compound is released by swelling and extrusion of the core components out through a passageway in a coating or surrounding shell or outer layer.

A first class includes matrix systems, in which a growth hormone secretagogue (GHSEC) is dissolved, embedded or dispersed in a matrix of another material that serves to retard the release of the GHSEC into an aqueous environment [e.g., the lumenal fluid of the gastrointestinal tract (GI)]. When a GHSEC is dissolved, embedded or dispersed in a matrix of this sort, release of the active compound takes place principally from the surface of the matrix. Thus, the GHSEC is released from the surface of a device which incorporates the matrix after it diffuses through the matrix into the surrounding fluid or when the surface of the device dissolves or erodes, exposing the active compound. In some embodiments, both mechanisms can operate simultaneously. The matrix systems may be large, i.e., tablet sized (about 1 cm), or small (< 0.3 cm). The system may be unitary, it may be divided by virtue of being composed of several sub-units (for example, several tablets which constitute a single dose) which are administered substantially simultaneously, it may consist of several small tablets within a capsule, or it may comprise a plurality of particles, referred to herein as a multiparticulate. A multiparticulate can have numerous formulation applications. For example, a multiparticulate may be used as small beads or as powder for filling a capsule shell, it may be compressed into a tablet, or it may be used *per se* for mixing with food (for example, ice cream) to increase palatability, or as a sachet that may be dispersed in a liquid, such as fruit juice or water.

The multiplicity of variables affecting release of a GHSEC from matrix devices permits abundant flexibility in the design of devices of different materials, sizes, and release times.

Non-eroding matrix tablets that provide sustained release of a GHSEC can be made with a GHSEC and water insoluble materials such as waxes, cellulose, or other water insoluble polymers. Matrix materials useful for the manufacture of these dosage forms include microcrystalline cellulose such as Avicel® (FMC Corp., Philadelphia, PA), including grades of microcrystalline cellulose to which binders such as hydroxypropyl methyl cellulose have been added, waxes such as paraffin, modified vegetable oils, carnauba wax, hydrogenated castor oil, beeswax, and the like, as well as polymers such as cellulose, cellulose esters, cellulose ethers, poly(vinyl chloride), poly(vinyl acetate), copolymers of vinyl acetate and ethylene, polystyrene, and the like. Water soluble binders or release modifying agents which can optionally be formulated into the matrix include water-soluble polymers such as hydroxypropyl cellulose (HPC), hydroxypropyl methyl cellulose (HPMC), methyl cellulose, poly (N-vinyl-2-pyrrolidinone) (PVP), poly(ethylene oxide) (PEO), poly(vinyl alcohol) (PVA), xanthan gum, carrageenan, and other such natural and synthetic materials. In addition, materials that function as release-modifying agents include water-soluble materials such as sugars or salts. Preferred water-soluble materials include lactose, sucrose, glucose, and mannitol, as well as HPC, HPMC, and PVP. In addition, solubilizing acid excipients such as organic acids including but not limited to malic acid, citric acid, erythorbic acid, ascorbic acid, adipic acid, glutamic acid, maleic acid, aconitic acid, fumaric acid, succinic acid, tartaric acid, and aspartic acid and solubilizing excipients such as sodium bitartrate and cyclodextrins, can be incorporated into matrix tablets to increase the release rate of the GHSEC, increase the total quantity of the GHSEC released, and potentially increase absorption and consequently the bioavailability of the GHSEC, particularly from matrix formulations that release the GHSEC over a period of six hours or longer.

In addition to components of the matrix system, the size of the matrix system can affect the rate of GHSEC release; therefore, a large matrix system such as a tablet will, in general, have a different composition from a small one such as a multiparticulate to achieve similar release profiles. The effect of the size of the matrix system on the kinetics of GHSEC release follows scaling behavior well known to those skilled in the art. By way of illustration, the following table shows the diffusion coefficient of a GHSEC through the matrix required to achieve a characteristic time for release of 10 hours for matrix systems of different sizes that release a GHSEC by a diffusive-based mechanism (rather than an eroding or in combination with an eroding mechanism).

| radius (cm) | diffusion coefficient (cm²/sec) |
|---|---|
| 0.0025 (50µm diameter) | 1.7 x 10⁻¹⁰ |
| 0.1 (2mm diameter) | 3 x 10⁻⁷ |
| 0.5 (1cm diameter) | 7 x 10⁻⁶ |

The above table illustrates that diffusion coefficients necessary to achieve the target characteristic time of release can change by orders of magnitude as the desired size of the device changes. Matrix materials that can be used to provide a GHSEC diffusion coefficient at the low end of the diffusion coefficient scale are polymers such as cellulose acetate. Conversely, materials at the upper end of the scale are materials such as polymers that form hydrogels or a water-swollen mass when hydrated. The rate of diffusion for any particular device can accordingly be tailored by the material or materials selected and the structure of the matrix.

For purposes of further illustration, to obtain a sustained release non-eroding matrix in a particle of about 50 µm in diameter, a matrix material of a polymer such as cellulose acetate or a similar material will likely be required, the slow diffusing matrix material tending to offset the short distances characteristic of small particle size. In contrast, in order to obtain sustained release in a large (e.g., 1 cm) device, a material which is more liquid-like (e.g., a hydrogel or water-soluble polymer) or with greater porosity will likely be required. For devices of an intermediate size, e.g., about 1 mm in diameter, a matrix composition of intermediate characteristics can be employed.

It is also noted that the effective diffusion coefficient of a GHSEC in a matrix may be increased to the desired value by the addition of plasticizers, pores, or pore-inducing additives, as known in the art. Slowly hydrating materials may also be used to effectively reduce the diffusion rates of a GHSEC, particularly at times shortly after administration. In addition to changing the effective diffusion coefficient, the release rate can also be altered by the inclusion of more soluble salt forms of the GHSEC (relative to the free base form) or excipients such as acids that solubilize the GHSEC.

A further sustained release non-eroding matrix system comprises a GHSEC dispersed in a hydrogel matrix. This embodiment differs from the hydrophilic matrix tablet in that the hydrogel of this embodiment is not a compressed tablet of soluble or erodible granular material, but rather is a monolithic polymer network. As is known in the art, a hydrogel is a water-swellable network polymer. Hydrogels can be made in many geometries, such as caplets, tablets, and multiparticulates. As an example, tablets can be prepared by standard techniques containing 10 to 80% of a crosslinkable polymer. Once tablets are formed the polymer can be crosslinked via a chemical crosslinking agent such as gluteraldehyde or via UV irradiation forming a hydrogel matrix. Hydrogels are preferred materials for matrix devices because they can absorb or be made to contain a large volume fraction of water, thereby permitting diffusion of solvated active compound within the matrix. Diffusion coefficients of active compounds in hydrogels are characteristically high, and for highly water-swollen gels, the diffusion coefficient of the active compound in the gel may approach the value in pure water. This high diffusion coefficient permits practical release rates from relatively large devices (i.e., it is not necessary to form microparticles). Although hydrogel devices can be prepared, loaded with a GHSEC, stored, dispensed and dosed in the fully hydrated state, it is preferred that they be stored, dispensed, and dosed in a dry state. In addition to stability and convenience, dry state dosing of hydrogel devices can provide good GHSEC release kinetics due to Case II transport (i.e., combination of swelling of hydrogel and diffusion of active compound out through the swollen hydrogel). Preferred materials for forming hydrogels include hydrophilic vinyl and acrylic polymers, polysaccharides such as calcium alginate, and poly(ethylene oxide). Especially preferred are poly(2-hydroxyethyl methacrylate), poly(acrylic acid), poly(methacrylic acid), poly(N-vinyl-2-pyrolidinone), poly(vinyl alcohol) and their copolymers with each other and with hydrophobic monomers such as methyl methacrylate, vinyl acetate, and the like. Also preferred are hydrophilic polyurethanes containing large poly(ethylene oxide) blocks. Other preferred materials include hydrogels comprising interpenetrating networks of polymers, which may be formed by addition or by condensation polymerization, the components of which may comprise hydrophilic and hydrophobic monomers such as those just enumerated.

Non-eroding matrix tablets can be made by tabletting methods common in the pharmaceutical industry. Preferred embodiments of non-eroding matrix tablets contain about 1 to about 80% GHSEC, about 5 to about 50% insoluble matrix materials such as cellulose, cellulose acetate, or ethylcellulose, and optionally about 5 to about 85% plasticizers, pore formers or solubilizing excipients, and optionally about 0.25 to about 2% of a tabletting lubricant, such as magnesium stearate, sodium stearyl fumarate, zinc stearate, calcium stearate, stearic acid, polyethyleneglycol-8000, talc, or mixtures of magnesium stearate with sodium lauryl sulfate. These materials can be blended, granulated, and tabletted using a variety of equipment common to the pharmaceutical industry.

A non-eroding matrix multiparticulate comprises a plurality of GHSEC-containing particles, each particle comprising a mixture of GHSEC with one or more excipients selected to form a matrix capable of limiting the dissolution rate of the GHSEC into an aqueous medium. The matrix materials useful for this embodiment are generally water-insoluble materials such as triglycerides, waxes, cellulose, or other water-insoluble polymers. If needed, the matrix materials may optionally be formulated with water-soluble materials that can be used as binders or as permeability-modifying agents. Matrix materials useful for the manufacture of these dosage forms include microcrystalline cellulose such as Avicel® (FMC Corp., Philadelphia, PA), including grades of microcrystalline cellulose to which binders such as hydroxypropyl methyl cellulose have been added, waxes such as paraffin, modified vegetable oils, camauba wax, hydrogenated castor oil, beeswax, and the like, as well as synthetic polymers such as poly(vinyl chloride), poly(vinyl acetate), copolymers of vinyl acetate and ethylene, polystyrene, and the like. Water soluble release modifying agents that can optionally be formulated into the matrix include water-soluble polymers such as HPC, HPMC, methyl cellulose, PVP, PEO, PVA, xanthan gum, carrageenan, and other such natural and synthetic materials. In addition, materials that function as release-modifying agents include water-soluble materials such as sugars or salts. Preferred water-soluble materials include lactose, sucrose, glucose, and mannitol, as well as HPC, HPMC, and PVP. In addition, any of the solubilizing acids or excipients previously mentioned can be incorporated into matrix multiparticulates to increase the release rate of the GHSEC, increase the total quantity of the GHSEC released, and potentially increase absorption and consequently the bioavailability of the GHSEC, particularly from matrix formulations that release the GHSEC over a period of six hours or longer.

A preferred process for manufacturing matrix multiparticulates is the extrusion/spheronization process. For this process, the GHSEC is wet-massed with a binder, extruded through a perforated plate or die, and placed on a rotating disk. The extrudate ideally breaks into pieces, which are rounded into spheres, spheroids, or rounded rods on the rotating plate. A preferred process and composition for this method involves using water to wet-mass a blend comprising about 20 to about 99% of microcrystalline cellulose blended with, correspondingly, about 80 to about 1% GHSEC.

A preferred process for manufacturing matrix multiparticulates is the rotary granulation process. For this process the GHSEC and excipients such as microcrystalline cellulose are placed in a rotor bowl in a fluid-bed processor. The active compound and excipient are fluidized, while spraying a solution that binds the active compound and excipients together in granules or multiparticulates. The solution sprayed into the fluid bed can be water or aqueous solutions or suspensions of binding agents such as polyvinylpyrrolidone or hydroxypropylmethylcellulose. A preferred composition for this method can comprise about 1 to about 80% GHSEC, about 10 to about 60% microcrystalline cellulose, and about 0 to about 25% binding agent.

A further preferred process for manufacturing matrix multiparticulates involves coating the GHSEC, matrix-forming excipients, and if desired, release-modifying or solubilizing excipients onto seed cores such as sugar seed cores known as non-pareils. Such coatings can be applied by many methods known in the pharmaceutical industry, such as spray-coating in a fluid bed coater, spray-drying, and granulation methods such as fluid bed or rotary granulation. Coatings can be applied from aqueous, organic or melt solutions or suspensions.

A further preferred process for manufacturing matrix multiparticulates is the preparation of wax granules via a melt-congeal process. In this process, a desired amount of the GHSEC is stirred with liquid wax to form a homogeneous mixture, cooled and then forced through a screen to form granules. Alternatively, the homogeneous mixture can be fed to a spinning disc where the mixture is broken up into droplets as it is spun off the edges of the disc. These droplets are then cooled, and solidify before landing in a collection chamber. Preferred matrix materials are waxy substances. Especially preferred are hydrogenated castor oil, glyceryl behenate, microcrystalline wax, camauba wax, and stearyl alcohol.

A further preferred process for manufacturing matrix multiparticulates involves using an organic solvent to aid mixing of the GHSEC with the matrix material. This technique can be used when it is desired to utilize a matrix material with an unsuitably high melting point that, if the material were employed in a molten state, would cause decomposition of the active compound or of the matrix material, or would result in an unacceptable melt viscosity, thereby preventing mixing of the GHSEC with the matrix material. GHSEC and matrix material may be combined with a modest amount of solvent to form a paste, and then forced through a screen to form granules from which the solvent is then removed. Alternatively, the GHSEC and matrix material may be combined with enough solvent to completely dissolve the matrix material and the resulting solution (which may contain solid active compound particles) spray dried to form the particulate dosage form. This technique is preferred when the matrix material is a high molecular weight synthetic polymer such as a cellulose ether or cellulose ester. Solvents typically employed for the process include acetone, ethanol, isopropanol, ethyl acetate, and mixtures of two or more.

A further process for manufacturing matrix multiparticulates involves using an aqueous solution or suspension of the GHSEC and matrix forming materials. The solution or suspension can be spray dried or sprayed or dripped into a quench bath or through a light chamber to initiate crosslinking of matrix materials and solidify the droplets. In this manner matrices can be made from latexes (e.g., dispersed ethyl cellulose with a plasticizer such as oleic acid or with a volatile water miscible solvent such as acetone or ethanol) by spray-drying techniques. Matrices can also be made in this manner by crosslinking a water soluble polymer or gum. For example, sodium alginate can be crosslinked by spraying into a solution containing soluble calcium salts, polyvinyl alcohol can be crosslinked by spraying into a solution containing gluteraldehyde, and di- and tri-acrylates can be crosslinked by UV irradiation.

Once formed, GHSEC matrix multiparticulates may be blended with compressible excipients such as lactose, mannitol, microcrystalline cellulose, dicalcium phosphate, and the like and the blend compressed to form a tablet. Disintegrants such as sodium starch glycolate, sodium croscarmellose, or crosslinked poly(vinyl pyrrolidone) are also usefully employed. Tablets prepared by this method disintegrate when placed in an aqueous medium (such as the GI tract), thereby exposing the multiparticulate matrix, which releases the GHSEC. GHSEC matrix multiparticulates may also be filled into capsules, such as hard gelatin capsules. Multiparticulates can also be directly dosed as a sachet that is mixed with water or other suitable drink, or can be sprinkled directly on food.

A further embodiment of a matrix system has the form of a hydrophilic matrix tablet containing a GHSEC that eventually dissolves or disperses in water and an amount of hydrophilic polymer sufficient to provide a useful degree of control over the release of GHSEC. GHSEC can be released from such matrices by diffusion, erosion or dissolution of the matrix, or a combination of these mechanisms. Hydrophilic polymers useful for forming a hydrophilic matrix include HPMC, HPC, hydroxy ethyl cellulose (HEC), PEO, PVA, polyacrylic acid, xanthan gum, carbomer, carrageenan, and zooglan. A preferred material is HPMC. Other similar hydrophilic polymers may also be employed. In use, the hydrophilic material is swollen by, and eventually dissolves or disperses in, water. The GHSEC release rate from hydrophilic matrix formulations may be controlled by the amount and molecular weight of hydrophilic polymer employed. In general, using a greater amount of the hydrophilic polymer decreases the release rate, as does using a higher molecular weight polymer. Using a lower molecular weight polymer increases the release rate. The release rate may also be controlled by the use of water-soluble additives such as sugars, salts, or soluble polymers. Examples of these additives are sugars such as lactose, sucrose, or mannitol, salts such as NaCI, KCI, NaHCO₃, and water soluble polymers such as PVP, low molecular weight HPC or HMPC or methyl cellulose. In general, increasing the fraction of soluble material in the formulation increases the release rate. In addition, any of the solubilizing acid excipients previously mentioned can be incorporated into matrix tablets to increase the release rate of GHSEC, increase the total quantity of GHSEC released, and potentially increase absorption and consequently the bioavailability of GHSEC, particularly from matrix formulations that release GHSEC over a period of six hours or longer. A hydrophilic matrix tablet typically comprises about 1 to about 90% by weight of the GHSEC and about 80 to about 10% by weight of polymer.

A preferred hydrophilic matrix tablet comprises, by weight, about 3% to about 80% GHSEC, about 5% to about 35% HPMC, about 0% to about 55% lactose or mannitol, about 0% to about 15% PVP, about 0% to about 20% microcrystalline cellulose, and about 0.25% to about 2% magnesium stearate.

Mixtures of polymers and/or gums can also be utilized to make hydrophilic matrix systems. For example, homopolysaccharide gums such as galactomannans (e.g., locust bean gum or guar gum) mixed with heteropolysaccharide gums (e.g., xanthan gum or its derivatives) can provide a synergistic effect that in operation provides faster forming and more rigid matrices for the release of active compound (See, for example, U.S. patents 5,455,046 and 5,512,297). Optionally, crosslinking agents such as calcium salts can be added to improve matrix properties.

Hydrophilic matrix formulations that eventually dissolve or disperse can also be made in the form of multiparticulates. Hydrophilic matrix multiparticulates can be manufactured by the techniques described previously for non-eroding matrix multiparticulates. Preferred methods of manufacture are layering GHSEC, a hydrophilic matrix material, and if desired release modifying agents onto seed cores (e.g., non-pareils) via a spray-coating process or forming multiparticulates by granulation, such as by rotary granulation of GHSEC, hydrophilic matrix material, and if desired, release modifying agents.

The matrix systems as a class often exhibit non-constant release of the active compound from the matrix. This result may be a consequence of the diffusive mechanism of active compound release, and modifications to the geometry of the dosage form and/or coating or partially coating the dosage form can be used to advantage to make the release rate of the active compound more constant as detailed below.

In a further embodiment, a GHSEC matrix tablet is coated with an impermeable coating, and an orifice (for example, a circular hole or a rectangular opening) is provided by which the content of the tablet is exposed to the aqueous GI tract. These embodiments are along the lines of those presented in U.S. 4,792,448 to Ranade, and as described by Hansson *et al*., *J. Pharm. Sci.,* **77** (1988) 322-324. The opening is typically of a size such that the area of the exposed underlying GHSEC constitutes less than about 40% of the surface area of the device, preferably less than about 15%.

In another embodiment, a GHSEC matrix tablet is coated with an impermeable material on part of its surface, e.g., on one or both tablet faces, or on the tablet radial surface.

In another embodiment, a GHSEC matrix tablet is coated with an impermeable material and an opening for active compound transport produced by drilling a hole through the coating. The hole may be through the coating only, or may extend as a passageway into the tablet.

In another embodiment, a GHSEC matrix tablet is coated with an impermeable material and a passageway for active compound transport produced by drilling a passageway through the entire tablet.

In another embodiment, a GHSEC matrix tablet is coated with an impermeable material and one or more passageways for active compound transport are produced by removing one or more strips from the impermeable coating or by cutting one or more slits through the coating, preferably on the radial surface or land of the tablet.

In another embodiment, a GHSEC matrix tablet is shaped in the form of a cone and completely coated with an impermeable material. A passageway for active compound transport is produced by cutting off the tip of the cone.

In another embodiment, a GHSEC matrix tablet is shaped in the form of a hemisphere and completely coated with an impermeable material. A passageway for active compound transport is produced by drilling a hole in the center of the flat face of the hemisphere.

In another embodiment, a GHSEC matrix tablet is shaped in the form of a half-cylinder and completely coated with an impermeable material. A passageway for GHSEC transport is produced by cutting a slit through (or removing a strip from) the impermeable coating along the axis of the half-cylinder along the centerline of the flat face of the half-cylinder. Those skilled in the art will appreciate that the geometric modifications to the embodiments described above can be equivalently produced by more than one method.

By "impermeable material" is meant a material having sufficient thickness and impermeability to GHSEC such that the majority of GHSEC is released through the passageway rather than through the "impermeable material" during the time scale of the intended active compound release. Such a coating can be obtained by selecting a coating material with a sufficiently low diffusion coefficient for GHSEC and applying it sufficiently thickly. Materials for forming the impermeable coating of these embodiments include substantially all materials in which the diffusion coefficient of the GHSEC is less than about 10⁻⁷ cm²/sec. It is noted that the preceding diffusion coefficient can be amply sufficient to allow release of GHSEC from a matrix device, as discussed above. However, for a device of the type now under discussion that has been provided with a macroscopic opening or passageway, a material with this diffusion coefficient is effectively impermeable to GHSEC relative to GHSEC transport through the passageway. Preferred coating materials include film-forming polymers and waxes. Especially preferred are thermoplastic polymers, such as poly(ethylene-co-vinyl acetate), poly(vinyl chloride), ethylcellulose, and cellulose acetate. These materials exhibit the desired low permeation rate of GHSEC when applied as coatings of thickness greater than about 100 µm.

A second class of GHSEC sustained-release dosage forms of the present invention includes membrane-moderated or reservoir systems such as membrane-coated diffusion-based capsule, tablet, or multiparticulate. Capsules, tablets and mutiparticulates can all be reservoir systems, such as membrane-coated diffusion-based. In this class, a reservoir of GHSEC is surrounded by a rate-limiting membrane. The GHSEC traverses the membrane by mass transport mechanisms well known in the art, including but not limited to dissolution in the membrane followed by diffusion across the membrane or diffusion through liquid-filled pores within the membrane. These individual reservoir system dosage forms may be large, as in the case of a tablet containing a single large reservoir, or multiparticulate, as in the case of a capsule containing a plurality of reservoir particles, each individually coated with a membrane. The coating can be non-porous, yet permeable to GHSEC (for example, GHSEC may diffuse directly through the membrane), or it may be porous.

Sustained release coatings as known in the art may be employed to fabricate the membrane, especially polymer coatings, such as a cellulose ester or ether, an acrylic polymer, or a mixture of polymers. Preferred materials include ethyl cellulose, cellulose acetate and cellulose acetate butyrate. The polymer may be applied as a solution in an organic solvent or as an aqueous dispersion or latex. The coating operation may be conducted in standard equipment such as a fluid bed coater, a Wurster coater, or a rotary bed coater.

If desired, the permeability of the coating may be adjusted by blending of two or more materials. A particularly useful process for tailoring the porosity of the coating comprises adding a pre-determined amount of a finely-divided water-soluble material, such as sugars or salts or water-soluble polymers to a solution or dispersion (e.g., an aqueous latex) of the membrane-forming polymer to be used. When the dosage form is ingested into the aqueous medium of the GI tract, these water soluble membrane additives are leached out of the membrane, leaving pores that facilitate release of the active compound. The membrane coating can also be modified by the addition of plasticizers, as known in the art.

A particularly useful variation of the process for applying a membrane coating comprises dissolving the coating polymer in a mixture of solvents chosen such that as the coating dries, a phase inversion takes place in the applied coating solution, resulting in a membrane with a porous structure. Numerous examples of this type of coating system are given in U.S. Patent 5,612,059.

The morphology of the membrane is not of critical importance so long as the permeability characteristics enumerated herein are met. However, specific membrane designs will have membrane morphology constraints in order to achieve the desired permeability. The membrane can be amorphous or crystalline. It can have any category of morphology produced by any particular process and can be, for example, an interfacially-polymerized membrane (which comprises a thin rate-limiting skin on a porous support), a porous hydrophilic membrane, a porous hydrophobic membrane, a hydrogel membrane, an ionic membrane, and other such membrane designs which are characterized by controlled permeability to GHSEC.

A useful reservoir system embodiment is a capsule having a shell comprising the material of the rate-limiting membrane, including any of the membrane materials previously discussed, and filled with a GHSEC active compound composition. A particular advantage of this configuration is that the capsule may be prepared independently of the active compound composition, thus process conditions that would adversely affect the active compound can be used to prepare the capsule. A preferred embodiment is a capsule having a shell made of a porous or a permeable polymer made by a thermal forming process. An especially preferred embodiment is a capsule shell in the form of an asymmetric membrane; i.e., a membrane that has a thin dense region on one surface and most of whose thickness is constituted of a highly permeable porous material. A preferred process for preparation of asymmetric membrane capsules comprises a solvent exchange phase inversion, wherein a solution of polymer, coated on a capsule-shaped mold, is induced to phase-separate by exchanging the solvent with a miscible non-solvent. Examples of asymmetric membranes useful in this invention are disclosed in U.S. Patents 5,698,220 and 5,612,059.

Tablets can also be reservoir systems. Tablet cores containing GHSEC can be made by a variety of techniques standard in the pharmaceutical industry. These cores can be coated with a rate-controlling coating as described above, which allows the GHSEC in the reservoir (tablet core) to diffuse out through the coating at the desired rate.

Another embodiment of reservoir systems comprises a multiparticulate wherein each particle is coated with a polymer designed to yield sustained release of GHSEC. The multiparticulate particles each comprise GHSEC and one or more excipients as needed for fabrication and performance. The size of individual particles, as previously mentioned, is generally between about 50 µm and about 3 mm, although beads of a size outside this range may also be useful. In general, the beads comprise GHSEC and one or more binders. As it is generally desirable to produce dosage forms that are small and easy to swallow, beads that contain a high fraction of GHSEC relative to excipients are preferred. Binders useful in fabrication of these beads include microcrystalline cellulose (e.g., Avicel® , FMC Corp.), HPC, HPMC, and related materials or combinations thereof. In general, binders that are useful in granulation and tabletting, such as starch, pregelatinized starch, and PVP may also be used to form multiparticulates.

Reservoir system GHSEC multiparticulates may be prepared using techniques known to those skilled in the art, including, but not limited to, the techniques of extrusion and spheronization, wet granulation, fluid bed granulation, melt-congealing, and rotary bed granulation. In addition, the beads may also be prepared by building the GHSEC composition (GHSEC plus excipients) up on a seed core (such as a non-pareil seed) by an active compound-layering technique such as powder coating or by applying the GHSEC composition by spraying a solution or dispersion of GHSEC in an appropriate binder solution onto seed cores in a fluidized bed such as a Wurster coater or a rotary processor. An example of a suitable composition and method is to spray a dispersion of a GHSEC/hydroxypropylcellulose composition in water.

A preferred method for manufacturing the multiparticulate cores of this embodiment is the extrusion/spheronization process, as previously discussed for matrix multiparticulates. A preferred process and composition for this method involves using water to wet-mass a blend of about 5 to about 99% of microcrystalline cellulose with correspondingly about 95 to about 1 % GHSEC. Especially preferred is the use of about 95 to about 50% microcrystalline cellulose with correspondingly about 5 to about 50% GHSEC.

A preferred process for making multiparticulate cores of this embodiment is the rotary-granulation process, as previously discussed for matrix multiparticulates.

Another preferred process for making multiparticulate cores of this embodiment is the melt-congeal process, as previously discussed for matrix multiparticulates.

Another preferred process for making multiparticulate cores of this embodiment is the process of coating seed cores with GHSEC and optionally other excipients, as previously discussed for matrix multiparticulates.

A sustained release coating as is known in the art, especially polymer coatings, may be employed to fabricate the membrane, as previously discussed for reservoir systems. Suitable and preferred polymer coating materials, equipment, and coating methods also include those previously discussed.

The rate of GHSEC release from the coated multiparticulates can also be controlled by factors such as the composition and binder content of the active compound-containing core, the thickness and permeability of the coating, and the surface-to-volume ratio of the multiparticulates. It will be appreciated by those skilled in the art that increasing the thickness of the coating will decrease the release rate, whereas increasing the permeability of the coating or the surface-to-volume ratio of the multiparticulates will increase the release rate. If desired, the permeability of the coating may be adjusted by blending of two or more materials. A useful series of coatings comprises mixtures of water-insoluble and water-soluble polymers, for example, ethylcellulose and hydroxypropyl methylcellulose, respectively. A particularly useful modification to the coating is the addition of finely-divided water-soluble material, such as sugars or salts. When placed in an aqueous medium, these water soluble membrane additives are leached out of the membrane, leaving pores that facilitate delivery of the active compound. The membrane coating may also be modified by the addition of plasticizers, as is known to those skilled in the art. A particularly useful variation of the membrane coating utilizes a mixture of solvents chosen such that as the coating dries, a phase inversion takes place in the applied coating solution, resulting in a membrane with a porous structure.

A preferred embodiment is a multiparticulate with cores comprising about 1 to about 50% GHSEC and about 10 to about 70% of one or more of the following: microcrystalline cellulose, lactose, mannitol, glyceryl behenate, stearyl alcohol, microcrystalline wax, PVP, HPC and HPMC. The individual cores are coated with either an aqueous dispersion of ethyl cellulose, which dries to form a continuous film, or a film of cellulose acetate containing PEG, sorbitol or glycerol as a release-modifying agent.

A third class of GHSEC sustained-release dosage forms includes the osmotic delivery devices or "osmotic pumps" as they are known in the art. Osmotic pumps comprise a core containing an osmotically effective composition surrounded by a semipermeable membrane. The term "semipermeable" in this context means that water can pass through the membrane, but solutes dissolved in the core permeate through the membrane at a rate significantly slower than water. In use, when placed in an aqueous environment, the device imbibes water due to the osmotic activity of the core composition. Owing to the semipermeable nature of the surrounding membrane, the contents of the device (including the active compound and any excipients) cannot pass through the non-porous regions of the membrane and are driven by osmotic pressure to leave the device through an opening or passageway pre-manufactured into the dosage form or, alternatively, formed *in situ* in the GI tract as by the bursting of intentionally-incorporated weak points in the coating under the influence of osmotic pressure, or alternatively, formed *in situ* in the GI tract by dissolution and removal of water-soluble porosigens incorporated in the coating. The osmotically effective composition includes water-soluble species that generate a colloidal osmotic pressure, and water-swellable polymers. The active compound itself (if highly water-soluble) may be an osmotically effective component of the mixture. 2-Amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate, having solubility in excess of 150 mg/ml, can provide an osmotic pressure of about 4 atmospheres, enough to contribute some osmotic driving force. Because this GHSEC is a base, its solubility is generally higher at acidic pH. Therefore, the osmotic effectiveness of the GHSEC is aided by presence of acidic buffers in the formulation. The active compound composition may be separated from the osmotically effective components by a movable partition or piston.

Materials useful for forming the semipermeable membrane include polyamides, polyesters, and cellulose derivatives. Preferred are cellulose ethers and esters. Especially preferred are cellulose acetate, cellulose acetate butyrate, and ethyl cellulose. Especially useful materials include those that spontaneously form one or more exit passageways, either during manufacturing or when placed in an environment of use. These preferred materials comprise porous polymers, the pores of which are formed by phase inversion during manufacturing, as described below, or by dissolution of a water-soluble component present in the membrane.

A class of materials that have particular utility for forming semipermeable membranes for use in osmotic delivery devices is that of porous hydrophobic polymers or vapor-permeable films, as disclosed by U.S. Patent 5,827,538. These materials are highly permeable to water, but highly impermeable to solutes dissolved in water. These materials owe their high water permeability to the presence of numerous microscopic pores (i.e., pores that are much larger than molecular dimensions). Despite their porosity, these materials are impermeable to molecules in aqueous solution because liquid water does not wet the pores. Water in the vapor phase is easily able to pass across membranes made from these materials. Such membranes are also known as vapor-permeable membranes.

A preferred embodiment of this class of osmotic delivery devices consists of a coated bi-layer tablet. The coating of such a tablet comprises a membrane permeable to water but substantially impermeable to GHSEC and excipients contained within. The coating contains one or more exit passageways in communication with the GHSEC-containing layer for delivering the GHSEC. The tablet core consists of two layers: one layer containing the GHSEC composition (including optional osmotic agents and hydrophilic water-soluble polymers) and another layer consisting of a water-swellable material, with or without additional osmotic agents.

When placed in an aqueous medium, the tablet imbibes water through the membrane, causing the GHSEC composition to form a dispensible aqueous composition, and causing the swellable layer to expand and push against the GHSEC composition, forcing the GHSEC composition out of the exit passageway. The GHSEC composition can swell aiding in forcing the GHSEC out the passageway. GHSEC can be delivered from this type of delivery system either dissolved or dispersed in the composition forced out of the exit passageway.

The rate of GHSEC delivery is controlled by such factors as the permeability and thickness of the coating, the osmotic pressure of the GHSEC-containing layer, the water activity of the swellable layer, and the surface area of the device. Those skilled in the art will appreciate that increasing the thickness of the coating will reduce the release rate, whereas increasing the permeability of the coating or the water activity of the hydrogel layer or the osmotic pressure of the GHSEC-containing layer or the surface area of the device will increase the release rate.

Exemplary materials that are useful to form the GHSEC composition, in addition to the GHSEC itself, include HPMC, PEO, and PVP, and other pharmaceutically-acceptable carriers. In addition, osmotic agents such as sugars or salts, especially sucrose, lactose, mannitol, or sodium bitartrate, may be added. Materials that are useful for forming the swelling layer include sodium carboxymethyl cellulose, poly(ethylene oxide), poly(acrylic acid), sodium (poly-acrylate), hydroxypropylmethylcellulose (HPMC), hydroxypropylcellulose (HPC), and other high molecular-weight hydrophilic materials. In addition, osmagents such as sugars or salts may be added. Particularly useful are poly(ethylene oxide)s having a molecular weight from about 5,000,000 to about 7,500,000.

Materials that are useful for forming the coating are cellulose esters, cellulose ethers, and cellulose ester-ethers. Preferred are cellulose acetate and ethylcellulose and optionally with PEG included as permeability modifying component.

The exit passageway must be located on the side of the tablet containing the GHSEC composition. There may be more than one such exit passageway. The exit passageway may be produced by mechanical means or by laser drilling, or by creating a difficult-to-coat region on the tablet by use of special tooling during tablet compression or by other means. The rate of GHSEC delivery from the device may be optimized so as to provide a method of delivering GHSEC to a mammal for optimum therapeutic effect.

Osmotic systems can also be made with a homogeneous core surrounded by a semipermeable membrane coating. GHSEC can be incorporated into a tablet core that also contains other excipients that provide sufficient osmotic driving force and optionally solubilizing excipients such as acids. A semipermeable membrane coating can be applied via conventional tablet-coating techniques such as using a pan coater. An active compound-delivery passageway can then be formed in this coating by drilling a hole in the coating, either by use of a laser or other mechanical means. Alternatively, the passageway may be formed by rupturing a portion of the coating or by creating a region on the tablet that is difficult to coat, as described above.

The core can consist of one or more pharmaceutically active compounds, water-soluble compounds for inducing osmosis, non-swelling solubilizing agents, non-swelling (water-soluble or water-insoluble) wicking agents, swellable hydrophilic polymers, binders and lubricants.

The osmotically active (water-soluble) agent is typically a sugar alcohol such as mannitol or sorbitol, or sugars in combination with polysaccharides such as dextrose and maltose, or a physiologically tolerable ionic salt that is compatible with the other components such as sodium or potassium chloride. Another osmotic agent is urea. Examples of water-soluble compounds for inducing osmosis are: inorganic salts such as magnesium chloride or magnesium sulfate, lithium, sodium or potassium chloride, lithium, sodium or potassium hydrogen or dihydrogen phosphate, salts of organic acids such as sodium or potassium acetate, magnesium succinate, sodium benzoate, sodium citrate or sodium ascorbate; carbohydrates such as sorbitol or mannitol (hexite), arabinose, dextrose, ribose or xylose (pentosene), glucose, fructose, galactose or mannose (hexosene), sucrose, maltose or lactose (disaccharides) or raffinose (trisaccharides); water-soluble amino acids such as glycine, leucine, alanine or methionine, urea and the like, and mixtures thereof. These water-soluble excipients may be present in the core in amounts by weight of about 0.01 to about 45%, based on the total weight of the therapeutic system.

Non-swelling solubilizing agents include (a) agents that inhibit crystal formation of the active agent or otherwise act by complexation therewith; (b) high HLB (hydrophilic-lipophilic balance) micelle-forming surfactants, particularly non-ionic and/or anionic surfactants: (c) citrate esters; and combinations thereof, particularly combinations of complexing agents and anionic surfactants. Examples of agents that inhibit crystal formation of the active agent or otherwise acts by complexation therewith include polyvinylpyrrolidone, polyethyleneglycol (particularly PEG 8000), cyclodextrins and modified cyclodextrins. Examples of high HLB, micelle forming surfactants include Tween 20, Tween 60, Tween 80, polyoxyethylene or polyethylene-containing surfactants, or other long chain anionic surfactants, particularly sodium lauryl sulfate. Examples of citrate ester derivatives that are preferred are the alkyl esters, particularly triethyl citrate. Combinations of these that are particularly preferred are polyvinylpyrrolidone with sodium lauryl sulfate and polyethyleneglycol with sodium lauryl sulfate.

Non-swelling wicking (wetting) agents are used to create channels or pores in the core of the tablet. This facilitates channeling of water through the core by physisorption. Preferred wicking agents do not swell to any appreciable degree. These materials can be water soluble or water insoluble materials. Water-soluble materials suitable for acting as wicking (wetting) agents include surface-active compounds, i.e., surfactants, e.g., anionic surfactants of the alkylsulfate type such as sodium, potassium or magnesium lauryl sulfate, n-tetradecylsulfate, n-hexadecyl sulfate or n-octadecylsulfate; of the alkyl ether sulfate type, e.g., sodium, potassium or magnesium n-dodecyloxyethyl sulfate, n-tetradecyloxyethyl sulfate, n-hexadecyloxyethyl sulfate or n-octadecyloxyethyl sulfate; or of the alkylsulfonate type, e.g. sodium potassium or magnesium n-dodecanesulfonate, n-tetradecanesulfonate, n-hexadecanesulfonate or n-octadecanesulfonate. Further suitable surfactants are nonionic surfactants of the fatty acid polyhydoxy alcohol ester type such as sorbitan monolaurate, sorbitan tristerate or triolate, polyethylene glycol fatty acid ester such as polyoxyethyl stearate, polyethylene glycol 400 stearate, polyethylene glycol 2000 stearate, preferably polyethylene oxide/propylene oxide block copolymers of the Pluronic® (BASF, Parsippany, NJ) or Synperonic® (ICI Surfactants, Everberg, Belgium) type, polyglycerol-fatty acid esters or glyceryl-fatty acid esters. Especially suitable is sodium lauryl sulfate. When present, these surfactants should be preferable present from about 0.2 to about 2% based on the total core weight. Other soluble wicking (wetting) agents include low molecular weight polyvinyl pyrrolidone and m-pyrol.

Insoluble materials suitable for acting as wicking (wetting) agents include, but are not limited to, colloidal silicon dioxide, kaolin, titanium dioxide, fumed silicon dioxide, alumina, niacinamide, bentonite, magnesium aluminum silicate, polyester, polyethylene. Particularly suitable insoluble wicking agents include colloidal silicon dioxide.

Suitable wall materials for forming the semi-permeable wall include microporous materials described in U.S. Patent. Nos. 3,916,899 and 3,977,404. It is possible to use acylated cellulose derivatives (cellulose esters) which are substituted by one to three acetyl groups or by one or two acetyl groups and a further acyl other than acetyl, e.g., cellulose acetate, cellulose triacetate, agar acetate, amylose acetate, beta glucan acetate, beta glucan triacetate, ethyl cellulose, cellulose acetate ethyl carbamate, cellulose acetate phthalate, cellulose acetate methyl carbamate, cellulose acetate succinate, cellulose acetate dimethylaminoaceate, cellulose acetate ethyl carbonate, cellulose acetate chloroacetate, cellulose acetate ethyl oxalate, cellulose acetate methylsulfonate, cellulose acetate butyl sulfonate, cellulose acetate propionate, cellulose acetate octate, cellulose acetate laurate, cellulose acetate p-toluenesulfonate, cellulose acetate butyrate, and other cellulose acetate derivatives. Suitable semi-permeable membrane materials are also triacetate of locust bean gum, methyl cellulose, hydroxypropyl methylcellulose and polymeric epoxides, copolymers of alkylene oxides, poly(vinyl methyl) ether polymers and alkyl glycidyl ethers, polyglycols or polylactic acid derivatives and further derivatives thereof. It is also possible to use mixtures of insoluble polymers, which when coated form a semi-permeable film, e.g. water insoluble acrylates, e.g., the copolymer of ethyl acrylate and methyl methacrylate.

A second, water-soluble component can be added to increase the permeability of the coating. Preferred water-soluble components are C₂-C₄ alkylene glycol, preferably polyethylene glycol.

An embodiment of GHSEC sustained release osmotic dosage forms of this invention comprises an osmotic GHSEC-containing tablet, which is surrounded by an asymmetric membrane, where said asymmetric membrane possesses one or more thin dense regions in addition to less dense porous regions. This type of membrane, similar to those used in the reverse-osmosis industry, generally allows higher osmotic fluxes of water than can be obtained with a dense membrane. When applied to a active compound formulation, e.g., a tablet, an asymmetric membrane allows high active compound fluxes and well-controlled sustained active compound release. This asymmetric membrane comprises a semipermeable polymeric material, that is, a material which is permeable to water, and substantially impermeable to salts and organic solutes such as a GHSEC.

Materials useful for forming the semipermeable membrane include polyamides, polyesters, and cellulose derivatives. Preferred are cellulose ethers and esters. Especially preferred are cellulose acetate, cellulose acetate butyrate, and ethyl cellulose. Especially useful materials include those which spontaneously form one or more exit passageways, either during manufacturing or when placed in an environment of use. These preferred materials comprise porous polymers, the pores of which are formed by phase inversion during manufacturing, as described above, or by dissolution of a water-soluble component present in the membrane.

The asymmetric membrane is formed by a phase-inversion process. The coating polymer, e.g., ethylcellulose or cellulose acetate, is dissolved in a mixed solvent system comprising a mixture of solvents (e.g., acetone) and non-solvents (e.g., water) for the ethylcellulose or cellulose acetate. The components of the mixed solvent are chosen such that the solvent (e.g., acetone) is more volatile than the non-solvent (e.g., water). When a tablet is dipped into such a solution, removed and dried, the solvent component of the solvent mixture evaporates more quickly than the non-solvent. This change in solvent composition during drying causes a phase-inversion, resulting in precipitation of the polymer on the tablet as a porous solid with a thin dense outer region. This outer region possesses multiple pores through which active compound delivery can occur.

In a preferred embodiment of an asymmetric membrane-coated tablet, the polymer/solvent/non-solvent mixture is sprayed onto a bed of tablets in a tablet-coating apparatus such as a Freund HCT-30 tablet coater (Freund Industrial Co., Tokyo, Japan).

In the environment of use, e.g., the GI tract, water is imbibed through the semipermeable asymmetric membrane into the tablet core. As soluble material in the tablet core dissolves, an osmotic pressure gradient across the membrane builds. When the hydrostatic pressure within the membrane enclosed core exceeds the pressure of the environment of use (e.g., the GI lumen), the GHSEC-containing solution is "pumped" out of the dosage form through preformed pores in the semipermeable membrane. The constant osmotic pressure difference across the membrane results in a constant well-controlled delivery of GHSEC to the use environment. A portion of the GHSEC dissolved in the tablet also exits via diffusion.

In this asymmetric-membrane-coated GHSEC tablet embodiment, high solubility salts of GHSEC are preferred. Also preferred are the inclusion of one or more solubilizing excipients, ascorbic acid, erythorbic acid, citric acid, fumaric acid, succinic acid, tartaric acid, sodium bitartrate, glutamic acid, aspartic acid, partial glycerides, glycerides, glyceride derivatives, polyethylene glycol esters, polypropylene glycol esters, polyhydric alcohol esters, polyoxyethylene ethers, sorbitan esters, polyoxyethylene sorbitan esters, saccharide esters, phospholipids, polyethylene oxide-polypropylene oxide block co-polymers, and polyethylene glycols. Most preferred are solubilizing excipients fumaric acid, ascorbic acid, succinic acid, and aspartic acid.

Osmotic tablets can also be made with a core tablet containing osmogents and/or solubilizing excipients surrounded first by a active compound containing layer and then second a semipermeable coating. The core tablet containing osmotic agents and/or solubilizing excipients can be made by standard tabletting methods known in the pharmaceutical industry. The semipermeable coating can then be applied to the layered core by many processes known in the art such as spray-coating or dip-coating methods described previously in these specifications. The active compound containing layer may be applied around the core by spray-coating methods where a solution or slurry of active compound and excipients is coated onto the tablet core. The active compound and excipients may also be layered around the tablet core by making a "layered" type of configuration using a tablet press to form a second active compound-containing layer around the tablet core. This type of compression coating method can be used to apply a powder coating (without solvents) around a tablet core.

Another embodiment of sustained release GHSEC osmotic dosage forms of this invention consists of GHSEC multiparticulates coated with an asymmetric membrane. GHSEC-containing multiparticulates are prepared by, for example, extrusion/spheronization or fluid bed granulation, or by coating non-pareil seeds with a mixture of GHSEC and a water-soluble polymer, as described above. GHSEC-containing multiparticulates are then spray-coated with a solution of a polymer in a mixture of a solvent and a non-solvent, as described above, to form asymmetric-membrane-coated multiparticulates. This spray-coating operation is preferably carried out in a fluid bed coating apparatus, e.g., a Glatt GPCG-5 fluid bed coater Glatt Air Techniques, Inc., Ramsey, NJ). The polymer used for forming the semipermeable asymmetric membrane is chosen as described above for asymmetric-membrane coated tablets. Likewise, excipients for the multiparticulate cores can be chosen as described above for asymmetric-membrane coated tablets.

Osmotic capsules can be made using the same or similar components to those described above for osmotic tablets and multiparticulates. The capsule shell or portion of the capsule shell can be semipermeable and made of materials described above. The capsule can then be filled either by a powder or liquid comprising GHSEC, excipients that provide osmotic potential, and optionally solubilizing excipients. The capsule core can also be made such that it has a bilayer or multilayer composition analogous to the bilayer tablet described above.

A fourth class of GHSEC sustained release dosage forms of this invention comprises coated swellable tablets and multiparticulates, as described in co-pending commonly assigned U.S. application no. 07/296,464, filed January 12, 1989 (published as EP 378404 A2; July 7, 1990). Coated swellable tablets comprise a tablet core comprising GHSEC and a swelling material, preferably a hydrophilic polymer, coated with a membrane that contains holes or pores through which, in the aqueous use environment, the hydrophilic polymer can extrude and carry out the GHSEC. Alternatively, the membrane may contain polymeric or low molecular weight water soluble porosigens which dissolve in the aqueous use environment, providing pores through which the hydrophilic polymer and GHSEC may extrude. Examples of porosigens are water-soluble polymers such as hydroxypropylmethylcellulose, and low molecular weight compounds like glycerol, sucrose, glucose, and sodium chloride. In addition, pores may be formed in the coating by drilling holes in the coating using a laser or other mechanical means. In this fourth class of GHSEC sustained release dosage forms, the membrane material may comprise any film-forming polymer, including polymers which are water permeable or impermeable, provided that the membrane deposited on the tablet core is porous or contains water-soluble porosigens or possesses a macroscopic hole for water ingress and GHSEC release. Multiparticulates (or beads) may be similarly prepared, with a GHSEC/swellable material core, coated by a porous or porosigen-containing membrane. Embodiments of this fourth class of GHSEC sustained release dosage forms may also be multilayered, as described in EP 378 404 A2.

Sustained release formulations may also be prepared with a portion of the dose released initially rapidly, followed by sustained release of the remaining portion of the dose.

Formulations that release a portion of the dose as a bolus shortly after administration and then release the remaining portion of the dose at a sustained release rate over time, such as over 2 hours to 18 hours or longer, can be made by a variety of methods. For example, a bilayer tablet can be formed with one layer having a sustained release matrix and the other layer an immediate release composition. Upon ingestion, the immediate release layer disintegrates leaving only the matrix tablet to provide sustained release. In another example, a drug coating can be applied over a matrix or osmotic tablet or over sustained release multiparticulates. The coating can be applied using typical coating equipment standard to the pharmaceutical industry. The active compound can either be a solution or in suspension and is typically mixed with a water soluble polymer in the coating solution. In addition, a combination dosage form can be made by mixing sustained release multiparticulates and immediate release multiparticulates in one dosage form. A preferred method of making a formulation that has an immediate release component and a controlled-release component is to apply a compression coating around an osmotic tablet.

Osmotic tablets comprise a tablet core that contains active compound and may contain excipients that have an osmotic potential greater than the fluid in the environment of use or contain water swellable materials. The tablet cores are surrounded by a semipermeable coating that allows water to be imbibed into the tablet core. In operation it is important that this semipermeable coating remain intact, if the coating is cracked or disrupted dose dumping could occur or the release rate could significantly increase. A compression coating is made by compressing a powder granulation around a tablet core to form a outer layer or coating. This is done in specialized tablet presses where the inner core is place in the powder/granulation during the compression step. Applying an immediate release active compound layer around an osmotic tablet core can be done without cracking or disrupting the semipermeable coating and thus, without affecting the release rate from the osmotic tablet within the compression coating.

In addition to sustained release dosage forms, the present invention contemplates delayed release dosage forms. A delayed release dosage form can operate by being sensitive to its use environment such that it delays releasing a growth hormone secretagogue until after it has passed into the small intestine. This type of delayed release dosage form is dependent on position along the GI tract, is independent of time, and is herein referred to as a "spatial" dosage form, or as exhibiting "spatially delayed release". After the dosage form has entered the small intestine, it can release a growth hormone secretagogue in immediate fashion, "immediate release" meaning that no component or means is implemented in the dosage form which would deliberately retard or slow down release once the delay period has ended. Examples of spatially delayed dosage forms are (1) pH-triggered dosage forms that delay release of a growth hormone secretagogue until the dosage form enters the environment of the small intestine, which is above pH 6.0, and (2) small intestinal enzyme-triggered dosage forms which delay release of a growth hormone secretagogue until a coating on the dosage form is altered by interaction with lipases, esterases, or proteases in the small intestinal lumen, as appropriate. In one aspect, spatially delayed dosage forms generally commence immediate release of a growth hormone secretagogue within approximately 30 minutes, preferably within 15 minutes, after passing out of the stomach into the small intestine. Alternatively, the spatially delayed dosage form can release growth hormone secretagogue over a period of time once the delay period has ended.

In addition to the spatially delayed release dosage forms discussed above, a dosage form can also operate by delaying the release of a growth hormone secretagogue for a set period of time. This type of dosage form is referred to herein as a "temporal" dosage form, or as exhibiting "temporally delayed release." A temporal delay is a delay occurring after the dosage form is ingested, which delay is not related to the spatial location of the dosage form in the gastrointestinal tract. Temporally delayed dosage forms may be considered to be triggered by the presence of water, and possess a means for delaying release of a growth hormone secretagogue for a specific time period after the dosage form enters an aqueous environment.

It is well known that the retention time of a dosage form in the stomach depends upon whether the subject has eaten. Certain dosage forms, e.g., non-disintegrating tablets, will remain in the stomach until the meal has substantially passed on into the duodenum, said gastric retention period lasting as long as three hours. Multiparticulate dosage forms will also spend a longer time in the fed stomach than in the fasted stomach, although in this case, the increased duration is reflected in a longer half-time for gastric emptying of these small multiparticulates.

A first spatially delayed release embodiment is a pH-dependent coated tablet, which comprises an immediate release tablet or tablet core coated with a material comprising a polymer that is substantially impermeable to a growth hormone secretagogue at the pH of the stomach, but which becomes permeable to a growth hormone secretagogue at the pH of the small intestine. "Substantially impermeable" in relation to spatially delayed dosage forms allows for very small amounts of a growth hormone secretagogue to be released through the coating, so long as not more than 10% of the growth hormone secretagogue contained in the dosage form is released in the stomach. Such polymers become permeable by virtue of dissolving or disintegrating or otherwise being disrupted so that a growth hormone secretagogue can freely pass through. The tablet or tablet core can comprise further excipients such as disintegrants, lubricants, fillers, and/or other conventional formulation ingredients. All such ingredients and/or excipients, regardless of the particular dosage form, are referred to herein collectively as the pharmaceutically acceptable "carrier". The core is coated with a material, preferably a polymer, which is substantially insoluble and impermeable at the pH of the stomach, but which is more permeable at the pH of the small intestine. Preferably, the coating polymer is substantially insoluble and impermeable at pH <5.0, and water-soluble or water-disintegrable at pH>5.0. Mixtures of a pH-sensitive polymer with a water-insoluble polymer may also be employed. pH-sensitive polymers that are relatively insoluble and impermeable at the pH of the stomach, but which are more soluble or disintegrabile or permeable at the pH of the small intestine and colon include polyacrylamides, phthalate derivatives such as acid phthalates of carbohydrates, amylose acetate phthalate, cellulose acetate phthalate, other cellulose ester phthalates, cellulose ether phthalates, hydroxypropylcellulose phthalate, hydroxypropylethylcellulose phthalate, hydroxypropylmethylcellulose phthalate, methylcellulose phthalate, polyvinyl acetate phthalate, polyvinyl acetate hydrogen phthalate, sodium cellulose acetate phthalate, starch acid phthalate, cellulose acetate trimellitate, styrene-maleic acid dibutyl phthalate copolymer, styrene-maleic acid polyvinylacetate phthalate copolymer, styrene and maleic acid copolymers, polyacrylic acid derivatives such as acrylic acid and acrylic ester copolymers, polymethacrylic acid and esters thereof, poly acrylic methacrylic acid copolymers, shellac, and vinyl acetate and crotonic acid copolymers.

Preferred pH-sensitive polymers include shellac, phthalate derivatives, particularly cellulose acetate phthalate, polyvinylacetate phthalate, and hydroxypropylmethylcellulose phthalate; cellulose acetate trimellitate; polyacrylic acid derivatives, particularly copolymers comprising acrylic acid and at least one acrylic acid ester; polymethyl methacrylate blended with acrylic acid and acrylic ester copolymers; and vinyl acetate and crotonic acid copolymers.

A particularly preferred group of pH-sensitive polymers includes cellulose acetate phthalate, polyvinylacetate phthalate, hydroxypropylmethylcellulose phthalate, anionic acrylic copolymers of methacrylic acid and methylmethacrylate, and copolymers comprising acrylic acid and at least one acrylic acid ester.

In a further embodiment of a spatially delayed dosage form, a "pH-dependent coated bead", beads 0.4 to 2.0 mm in diameter comprising growth hormone secretagogue plus carrier are coated with one or more of the aforementioned pH-sensive polymers. The coated beads may be placed in a capsule or may be compressed into a tablet, with care taken to avoid damaging the polymeric coat on individual beads during tablet compression. Preferred coated beads are those which exhibit essentially no release (i.e., less than10%) of the growth hormone secretagogue from the dosage form, as previously discussed, until the beads have exited the stomach, thus assuring that minimal growth hormone secretagogue is released in the stomach. The coating may comprise from 5% to 200% of the weight of the uncoated bead core. Preferably, the coating comprises from 10% to 100% of the weight of the bead core.

In a further embodiment of a multiparticulate spatially delayed growth hormone secretagogue dosage form, a "pH-dependent coated particle", the dosage form comprises small growth hormone secretagogue plus carrier particles from 0.1 to 0.4 mm in diameter. The particles are coated with one or more of the aforementioned pH-sensitive polymers. The coated particles may be used to make unit dose packs or may be placed in a capsule or may be compressed into a tablet, with care taken to avoid damaging the polymeric coat on individual particles during tablet compression. Preferred coated particles are those which exhibit essentially no release of growth hormone secretagogue from the dosage form (i.e., less than 10%) until the particles have exited the stomach, thus assuring that minimal growth hormone secretagogue is released in the stomach. Mixtures of a pH-sensitive polymer with a water-insoluble polymer are also included.

Growth hormone secretagogue-containing tablets and particles and beads may be coated with mixtures of polymers whose solubilities vary at different pH's. For example, preferred coatings comprise Eudragit® -L, or from 9:1 to 1:4 Eudragit® -L/Eudragit® -S (Rohm America, Inc., Piscataway, NJ).

A further embodiment of a spatially delayed growth hormone secretagogue dosage form constitutes a modification of the pH-dependent coated tablet, pH-dependent coated bead, and pH-dependent coated particle embodiments. The growth hormone secretagogue-containing core tablet, bead, or particle is first coated with a barrier coat, and then is coated with the pH-dependent coat. The function of the barrier coat is to separate the growth hormone secretagogue from the pH-dependent coat. A barrier coat prevents such premature release. Suitable barrier coatings are composed of water-soluble materials such as sugars such as sucrose, or water-soluble polymers such as hydroxypropyl cellulose, hydroxypropyl methylcellulose, and the like. Hydroxypropyl cellulose and hydroxypropylmethylcellulose and polyvinylpyrrolidone are preferred. The barrier coat may comprise from 1% to 20%, preferably from 2% to 15%, of the weight of the uncoated growth hormone secretagogue-containing tablet, bead or particle core.

In a further embodiment of a spatially delayed growth hormone secretagogue dosage form, a solution or suspension or powder of a growth hormone secretagogue in a solvent is encapsulated in a water soluble capsule, such as a hard or soft gelatin capsule as known in the art, and the capsule is coated with a pH-dependent polymer as described above for "pH-dependent coated tablets". In the preparation of growth hormone secretagogue solutions for encapsulation, solvents such as triglyceride oils and glycols may be used.

Preferred solvents are water-immiscible solvents including water-immiscible oils, including triglyceride vegetable oils such as safflower oil, sesame oil, olive oil, corn oil, castor oil, coconut oil, cottonseed oil, soybean oil, and the like. Also included are synthetic and semisynthetic medium chain triglyceride oils such as those sold under the tradename Miglyol® (HulsAmerica, Piscataway, New Jersey) or Captex® (Abitec Corp., Columbus, Ohio). Examples are triglycerides of caprylic/capric acids (Miglyol® -810, Miglyol® -812, Captex® -300, Captex® -355), and triglycerides of caprylic/capric/linoleic acids (Miglyol® -818). Also included are long chain triglyceride oils such as triolein, and other mixed chain triglycerides, which are liquid at room temperature.

Water-immiscible solvents also include monoglycerides and diglycerides such as those sold under the trademarks Capmul® (ABITEC, Columbus, Ohio) and Imwitor® (HulsAmerica, Piscataway, New Jersey). Examples are monoolein (Capmul® -GMO), mono and diglycerides of octanoic and decanoic acids (Imwitor® -742, Capmul® -MCM), and monooctanoin (Imwitor® -308), and the like.

Preferred oils are liquid at room temperature. Preferred mono-, di-, and triglycerides are those with an average acyl chain length of C₄-C₁₈.

Useful excipients further include various liquid esters of short chain alcohols, such as the propylene glycol ester of caprylic/capric acids (Miglyol® -840, Captex® -200). Fatty acids which are liquid at room or body temperature, such as caprylic acid, capric acid, lauric acid, oleic acid, or linoleic acid are also useful.

Further useful excipients include semisolid vehicles such as those sold under the registered trademark Gelucire® . Examples are PEG-32-glyceryl-laurate (Gelucire® 44/14), and glycerol esters of fatty acids (Gelucire® 33/01).

Further useful excipients also include surfactants and emulsifiers, which have the capacity to dissolve a growth hormone secretagogue. These surfactants and emulsifiers form micelles when they are mixed with aqueous media. Examples are polysorbate-80, nonylphenoxypolyoxyethylenes, dioctyl sodium sulfosuccinate, PEG-6 glyceryl mono-oleate (Labrafil® M-1944-CS), PEG-6 glyceryl linoleate (Labrafil® M-2125-CS), and the like.

Water-immiscible solvents may be mixed with surfactants and emulsifiers, in order to effect the spontaneous formation of small or microscopic vehicle droplets (e.g., microemulsions) when the water-immiscible solvent/emulsifier vehicle is mixed with water, as in the gastrointestinal tract. Such mixtures include mixtures of triglycerides, or mono- and di-glycerides, with polysorbates, e.g., mixtures of Capmul® -MCM and polysorbate-80, or mixtures of Miglyol® -812 and polysorbate-80, in ratios of from 99/1 to 50/50, respectively. Further useful mixtures include mixtures of mono-, di-, and triglycerides with polysorbates, e.g., Capmul® -MCM/Miglyol® -812/polysorbate-80, in which Capmul® -MCM makes up 40-80% of the vehicle, with the remainder being any combination of Miglyol® -812 and polysorbate-80. Further useful mixtures include a vegetable oil and a surfactant, e.g., olive oil/polysorbate-80 in ratios of 99:1 to 50:50, or corn oil/Labrafil® -M-2125-CS in ratios of 99:1 to 50:50. Polyethyleneglycols and other water-miscible growth hormone secretagogue solvents, e.g., glycerin, ethanol, propylene glycol, may be included in amounts up to 30% of the vehicle, in order to optimize growth hormone secretagogue solubility in the vehicle, or to improve the viscosity of the vehicle to aid in capsule filling.

Solutions of a growth hormone secretagogue in vehicles of the types described above are encapsulated in soft gelatin capsules, or are encapsulated in hard gelatin capsules. If encapsulated in hard gelatin capsules, it is preferred that the seam between the two capsule shell pieces be sealed, for example with a strip of gelatin to prevent leakage. Encapsulation in soft-gelatin is well known, and is described in "The Theory and Practice of Industrial Pharmacy", by L. Lachman, H. Lieberman, and J. Kanig, Lea and Febiger, publisher.

The pH-sensitive polymer may be any of those already disclosed, for example but not limited to cellulose acetate phthalate, polyvinylacetate phthalate, hydroxypropylmethylcellulose phthalate, copolymers of methacrylic acid and methylmethacrylate, and copolymers comprising acrylic acid and at least one acrylic acid ester.

Coating of growth hormone secretagogue-containing tablets, beads, capsules, and particles may be carried out using equipment known in the art. For example, growth hormone secretagogue-containing tablet cores and capsules may be coated with a pan-coater, such as a Hi-Coater (Freund Industrial Co.), or an Accela-Cota (Manesty Corp., Liverpool). Growth hormone secretagogue-containing beads and particles are preferably coated using a fluidized bed coater, such as a Wurster coater, utilizing coating equipment available for example from the Glatt Air Techniques, Inc. (Ramsey, NJ). Beads may also be coated using a rotary granulator, such as a CF-granulator available from Freund Industrial Co.

Advantageously, because pH-triggered spatially delayed devices possess a mechanism for sensing that the device has exited the stomach, interpatient variability in gastric emptying is not a significant issue.

In a further embodiment of a spatially delayed growth hormone secretagogue dosage form, an "enzyme-triggered supported liquid membrane device" comprises a growth hormone secretagogue formulated in a dosage form of the type described in WO 94/12159. This embodiment generally has the form of an immediate release tablet or multiparticulate (preferably a bead) containing a growth hormone secretagogue plus carrier, a microporous hydrophobic membrane that at least partially, preferably entirely, surrounds the tablet or bead, and a hydrophobic liquid entrained within the pores of the membrane. Alternatively, the growth hormone secretagogue plus carrier may be incorporated into a capsule shell which comprises a microporous hydrophobic membrane with a hydrophobic liquid entrained within the pores of the capsule shell. The hydrophobic liquid is substantially impermeable to both the aqueous environment and the growth hormone secretagogue tablet or bead core formulation. The hydrophobic liquid is capable of change such that it becomes permeable to the aqueous environment or growth hormone secretagogue formulation. After ingestion of this embodiment by a patient, growth hormone secretagogue release into the gastrointestinal system is delayed until the dosage form has exited the stomach and moved into the small intestine.

In a growth hormone secretagogue enzyme-triggered supported liquid membrane device, the entrained hydrophobic liquid is a liquid that undergoes change that is enzymatically catalyzed in the lumen of the small intestine, and not in the stomach, such that the pores become permeable to water and growth hormone secretagogue. The core can optionally contain an osmagent, swelling, or bursting material to help speed the release of growth hormone secretagogue once the dosage form has passed into the small intestine. Exemplary hydrophobic liquids are triglycerides, fatty anhydrides, fatty acid esters of cholesterol, hydrophobic amino acid esters, and the like. Preferred triglycerides include triolein, tricaprylin, trilaurin, olive oil, palm oil, coconut oil, sesame seed oil, corn oil, peanut oil, soybean oil, and the like. Preferred fatty acid anhydrides include caprylic anhydride, lauric anhydride, myristic anhydride and the like. Mixtures of hydrophobic liquids may be used. Exemplary materials for the microporous hydrophobic support membrane include cellulose esters, polycarbonates, polyalkenes, polystyrenes, polyvinyl esters, polysiloxanes, polyacrylates, and polyethers. Preferably, the hydrophobic microporous membrane with entrained hydrophobic liquid is impermeable to growth hormone secretagogue, until gastrointestinal enzymes have catalyzed a change in the hydrophobic oil, as described below.

In the environment of use, i.e., the small intestinal lumen, lipases and esterases degrade the aforementioned hydrophobic oils, releasing surfactant products in the pores of the microporous membrane of this embodiment, thus producing aqueous channels through which the growth hormone secretagogue in the device core may exit through the microporous hydrophobic support membrane. Release of the growth hormone secretagogue may occur by simple diffusion, osmotic pumping, osmotic bursting, or by bursting due to the presence of a swellable material, e.g., hydrogel, in the growth hormone secretagogue-containing core of the device.

In a growth hormone secretagogue enzyme-triggered supported liquid membrane device as disclosed above, hydrophobic oils may be used which are substrates for small intestinal proteases such as carboxypeptidase and chymotrypsin. Exemplary oils are hydrophobic esters of amino acid derivatives.

In a further embodiment of a spatially delayed growth hormone secretagogue dosage form, growth hormone secretagogue tablets, capsules, beads, or powders are coated with a coating that contains components that are enzymatically degraded by enzymes in the lumen of the small intestine, but not in the gastric lumen. The coating comprises waxes or triglycerides of natural or synthetic origin, which are solid at body temperature. In preferred embodiments, 2-20% of a material which is liquid at body temperature, and which is degraded by small intestinal enzymes (e.g., trypsin, chymotrypsin, elastase, lipase), is included. Suitable enzymatically-labile liquids are those described above for "enzyme triggered supported liquid membrane devices."

In an embodiment of a temporally delayed growth hormone secretagogue dosage form, a "bursting osmotic core device," a growth hormone secretagogue is incorporated in an osmotic bursting device which comprises a tablet core or bead core comprising a growth hormone secretagogue and one or more osmagents. Devices of this type have been generally disclosed in U.S. 3,952,741. Examples of osmagents are sugars such as glucose, sucrose, mannitol, lactose, and the like; and salts such as sodium chloride, potassium chloride, sodium carbonate, and the like; water-soluble acids such as tartaric acid, fumaric acid, and the like. The growth hormone secretagogue-containing tablet core or bead core is coated with a polymer that forms a semipermeable membrane, that is, a membrane that is permeable to water, but is impermeable to growth hormone secretagogue. Examples of polymers that provide a semipermeable membrane are cellulose acetate, cellulose acetate butyrate, and ethylcellulose, preferably cellulose acetate. A melt mixture of a polyethylene glycol, e.g., polyethylene glycol-6000, and a hydrogenated oil, e.g., hydrogenated castor oil, may be used as a coating, as described for isoniazid tablets by Yoshino (Capsugel Symposia Series; Current Status on Targeted Active compound Delivery to the Gastrointestinal Tract; 1993; pp.185-190). Preferred semipermeable coating materials are cellulose esters and cellulose ethers, polyacrylic acid derivatives such as polyacrylates and polyacrylate esters, and polyvinyl alcohols and polyalkenes such as ethylene vinyl alcohol copolymer. Especially preferred semipermeable coating materials are cellulose acetate and cellulose acetate butyrate.

When a coated tablet or bead of a bursting osmotic core embodiment is placed in an aqueous environment of use, water passes through the semipermeable membrane into the core, dissolving a portion of the growth hormone secretagogue and osmagent, generating a colloidal osmotic pressure which results in bursting of the semipermeable membrane and release of the growth hormone secretagogue into the aqueous environment. By choice of bead or tablet core size and geometry, identity and quantity of osmagent, and thickness of the semipermeable membrane, the time lag between placement of the dosage form into the aqueous environment of use and release of the enclosed growth hormone secretagogue may be tailored. It will be appreciated by those skilled in the art that increasing the surface-to-volume ratio of the dosage form, and increasing the osmotic activity of the osmagent serve to decrease the time lag, whereas increasing the thickness of the coating will increase the time lag.

A bursting osmotic core device possesses no mechanism for sensing that the device has exited the stomach and entered the small intestine. Thus, devices of this type are temporally delayed devices, that is, devices that release a growth hormone secretagogue at a predetermined time after entering an aqueous environment, i.e., after being swallowed. In the fasted state, indigestible non-disintegrating solids, such as the "bursting osmotic core devices" of this invention, are emptied from the stomach during phase III of the Interdigestive Migrating Myoelectric Complex (IMMC), which occurs approximately every 2 hours in the human. Depending on the stage of the IMMC at the time of dosing in the fasted state, a bursting osmotic core device may exit the stomach almost immediately after dosing, or as long as 2 hours after dosing. In the fed state, indigestible non-disintegrating solids, which are <11 mm in diameter, will empty slowly from the stomach with the contents of the meal (Khosla and Davis, Int. *J*. *Pharmaceut.* 62 (1990) R9-R11). If the indigestible non-disintegrating solid is greater than 11 mm in diameter, i.e., about the size of a typical tablet, it will be retained in the stomach for the duration of the digestion of the meal, and will exit from the stomach during phase III of an IMMC, after the entire meal has been digested and has exited the stomach.

In a further embodiment of a temporally delayed growth hormone secretagogue dosage form, a "bursting coated swelling core", a growth hormone secretagogue-containing tablet or bead is prepared that also comprises a swellable material, such as a swellable colloid (e.g., gelatin), as described in U.S. 3,247,066. Preferred swelling core materials are hydrogels, i.e., hydrophilic polymers which take up water and swell, such as polyethylene oxides, polyacrylic acid derivatives such as polymethyl methacrylate, polyacrylamides, polyvinyl alcohol, poly-N-vinyl-2-pyrrolidone, carboxymethylcellulose, starches, and the like. Preferred swelling hydrogels for this embodiment are polyethylene oxides, crosslinked polyacrylates, and carboxymethylcellulose. The colloid/hydrogel-containing growth hormone secretagogue-containing core tablet or bead is coated, at least in part, by a semipermeable membrane. Examples of polymers which provide a semipermeable membrane are cellulose acetate and cellulose acetate butyrate,and ethylcellulose. A melt mixture of a polyethylene glycol, e.g., polyethylene glycol-6000, and a hydrogenated oil, e.g., hydrogenated castor oil, may be used as a coating, as described for isoniazid tablets by Yoshino (Capsugel Symposia Series; Current Status on Targeted Active compound Delivery to the Gastrointestinal Tract; 1993; pp. 185-190). Preferred semipermeable coating materials are cellulose esters and cellulose ethers, polyacrylic acid derivatives such as polyacrylates and polyacrylate esters, and polyvinyl alcohols and polyalkenes such as ethylene vinyl alcohol copolymer. Especially preferred semipermeable coating materials are cellulose acetate and cellulose acetate butyrate.

When a coated tablet or bead having a bursting coated swelling core is placed in an aqueous environment of use, water passes through the semipermeable membrane into the core, swelling the core and resulting in bursting of the semipermeable membrane and release of a growth hormone secretagogue into the aqueous environment. By choice of bead or tablet core size and geometry, identity and quantity of swelling agent, and thickness of the semipermeable membrane, the time lag between placement of the dosage form into the aqueous environment of use and release of the enclosed growth hormone secretagogue may be chosen. Preferred bursting coated swelling core devices of this invention are those that exhibit substantially no release of growth hormone secretagogue from the dosage form until the dosage form has exited the stomach, thus assuring that minimal growth hormone secretagogue is released in the stomach.

A bursting coated swelling core device possesses no mechanism for sensing that the device has exited the stomach and entered the small intestine. Thus, devices of this type release their growth hormone secretagogue contents at a predetermined time after entering an aqueous environment, i.e., after being swallowed, as previously discussed for bursting osmotic core devices, and the same considerations and preferences apply to making bursting coated swelling core devices.

In a preferred embodiment of a temporally delayed growth hormone secretagogue dosage form, immediate release growth hormone secretagogue tablets, beads, or particles are prepared to serve as cores which are coated with a water-soluble and/or water-disintegrable delay layer. Preferred delay coatings comprise hydroxypropylcellulose (HPC), hydroxypropyl-methylcellulose (HPMC), polyethylene oxide, and polyvinyl pyrrolidone. For tablets this coating may be carried out in a tablet coating apparatus such as an HCT-30, HCT-60, or HCT-130 Coater (Freund Inc). The tablets are coated with an aqueous solution of HPMC or other appropriate polymer to a final coating weight of 5-50% of the final weight of the coated tablet. Heavier coating weights give longer delays before initiation of growth hormone secretagogue release into the use environment (the gastrointestinal lumen). The delay time may also be increased by incorporating small to moderate quantities of poorly water-soluble polymers (including but not limited to ethylcellulose (EC), cellulose acetate (CA), cellulose acetate butyrate) into the coating formulation. For example, the coating formulation may consist of 95:5 HPMC/EC to 50:50 HPMC/EC, or 95:5 HPMC/CA to 50:50 HPMC/CA. In the case of such mixed polymer coating systems, it may be necessary to adjust the solvent composition to dissolve the mixture of water-soluble and poorly water-soluble polymers. For example, mixtures of acetone and water, or ethanol and water, may be used as needed. Beads and particles may be similarly coated using a fluid bed coating apparatus, such as a Glatt GPCG-5 coater (Glatt Air Techniques, Inc). For beads, the coating comprises from 10% to 100% of the weight of the uncoated bead core.

In a further embodiment of a temporally delayed growth hormone secretagogue dosage form, a solution or suspension of a growth hormone secretagogue in a solvent is encapsulated in a soft or hard gelatin capsule that is then coated with a water-soluble and/or water-disintegratable polymer as described above for coating other types of cores. Useful and preferred solvents for a growth hormone secretagogue include all those mentioned above for use with spatially delayed encapsulated solutions. The coating comprises polymers such as hydroxypropylcellulose, hydroxypropylmethylcellulose, polyethylene oxide, polyvinyl pyrrolidone, cellulose acetate, and ethylcellulose.

It will be appreciated by those skilled in the art that the various coated growth hormone secretagogue tablet, bead, and particle embodiments described above can be coated using standard coating equipment, such as pan coaters (e.g., Hi-Coater available from Freund Industrial Co.; Accela-Cota available from Manesty, Liverpool), fluidized bed coaters, e.g., Wurster coaters, (available from Glatt Air Techniques, Inc., Ramsey, NJ and Aeromatic Corp., Columbia, MD), and rotary granulators, e.g., CF-Granulator (available from Freund Industrial Co). Core tablets are made on standard tablet presses, such as a Kilian press (Kilian and Company, Inc., Horsham, PA). Growth hormone secretagogue-containing beads and particles are made in fluidized bed granulators, rotary granulators, and extruder/spheronizers.

A preferred method of intermittent administration of a growth hormone secretagogue uses an immediate release formulation. It is also possible to intermittently administer a growth hormone secretagogue using a combination of an immediate release formulation and a sustained release formulation.

The patents, publications and any other documents cited herein are all hereby incorporated by reference. The clinical study detailed below is intended to illustrate particular embodiments of the invention and is not intended to limit the scope of the description, including the claims, in any way.

### Clinical Study

### Study Population:

This clinical study was performed in male and female subjects between the ages of 65 and 84 years inclusive, and whose baseline IGF-1 levels were <150 ng/ml.

### Dosing Regimen:

The study was a double blind, parallel group, placebo-controlled study.

In the first leg of the study, the safety and efficacy of 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate was studied in 4 groups of approximately 24 subjects at doses of 0 mg (placebo), 1 mg tid, 3 mg tid and 3 mg am and 6 mg pm. (All dosage forms in this part of the study were immediate release dosage forms.)

In addition, an extension study was conducted to evaluate the relationship between peak GH concentrations or IGF-I versus surrogate responses such as lipid concentrations and body composition. A controlled release (CR) formulation was also evaluated, either alone or combined with an immediate release (IR) formulation (24-30 patients per group). [16mg (10 CR, 6 IR) h.s.( h.s. means at bedtime); 16 mg (10 CR, 6 IR) h.s. every third day; 16 mg CR h.s.; and placebo.] The formulations are set forth below.

### Study Active Compound Administration:

In the extension study, 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate was supplied as 10 mg or 3 mg controlled release tablets and 3 mg immediate release tablets, with matching placebo tablets. Study active compound was supplied in blisterpaks, with five tablets to be taken at time of each dosing. Subjects were instructed to take the tablets with one glass of water at bedtime.

### Plan and Design:

Return visits were scheduled at 1, 2, and 4 weeks after baseline. A blood sample for 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate and IGF-1 levels was obtained at each visit. The baseline and all subsequent follow-up visits were performed at the same time of day (between approximately 8-11 am).

In addition to this schedule, subjects at selected sites in the extension study were studied during two overnight admissions on the first night of dosing and again on day 28 of dosing. Growth hormone secretion and pharmacokinetic sampling was carried out.

### Follow-up Period and End of Study:

A follow-up evaluation was made at 1, 2, and 4 weeks. Vital signs were obtained and the skin of the face, trunk and upper extremities was inspected. A blood sample was obtained for measurement of GH and IGF-1, and for measurement of plasma concentrations of 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate.

In addition, subjects at selected study centers were admitted to an overnight unit on day 28 of dosing. Growth hormone secretion profile, pharmacokinetics, and vital sign determination were carried out.

### Efficacy Endpoints:

The primary efficacy endpoint was the percentage change from baseline in IGF-1. Secondary outcomes included insulin-like growth factor binding protein 3 (IGFBP-3), cholesterol subfractions, and percent changes in total adipose and total lean tissue. Changes in each of the secondary efficacy measures over time were examined systematically either graphically or in tabular form and summarized using appropriate descriptive statistics as is well known in the art.

### Results:

In the first leg of the study, there was a dose related increase in IGF-I, with both groups administered 9 mg daily having similar increases of approximately 35%. IGFBP3 also increased. There were small changes in body composition consistent with increased GH secretion, i.e., decreased adipose tissue and increased apparent whole body lean mass.

In the extension study, there were increases in IGF-I in each of the 2 groups in which 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide L-tartrate was dosed daily. The group randomized to receive 2-amino-N-[2-(3aR-benzyl-2-methyl-3-oxo-2,3,3a,4,6,7-hexahydro-pyrazolo[4,3-c]pyridin-5-yl)-1R-benzyloxymethyl-2-oxo-ethyl]-isobutyramide L-tartrate every third day had a minimal average rise of IGF-I of approximately 10%. The group receiving CR alone had modest and sporadic GH peaks averaging less than 4 ng/ml at baseline and 2 ng/ml after 4 weeks. The groups receiving 6 mg IR (with 10 mg CR) had GH peaks at baseline averaging 15 ng/ml or more, which by 4 weeks had attenuated to 4-6 ng/ml. By a variety of analyses, there was less attenuation in the group receiving Q 3 day 2-amino-N-[2-(3a-(R)-benzyl-2-methyl-3-oxo-2,3,3a,4,6,7-hexahydro-pyrazolo[4,3-c]pyridin-5-yl)-1 R-benzyloxymethyl-2-oxo-ethyl]-isobutyramide L-tartrate. The Q 3 day group had peak heights on average 1.52 ng/ml greater than the QD group. Changes in body composition were again observed and were similar in the two groups receiving IR formulation despite large differences in the increase of IGF-I. Increases in lean tissue approximated 0.5-0.6% with corresponding reductions in adipose tissue.

The specific dosage forms used in the clinical study are set forth below wherein the active compound is 2-amino-N-[2-(3a-(R)-benzyl-2-methyl-3-oxo-2,3,3a,4,6,7-hexahydro-pyrazolo[4,3-c]pyridin-5-yl)-1R-benzyloxymethyl-2-oxo-ethyl]-isobutyramide L-tartrate.

| 1 mg immediate release tablet | | |
|---|---|---|
| Component | Grade of Component | Weight (mg/tablet) |
| Active compound | Pharmaceutical | 1.30^{(a)} |
| Calcium phosphate dibasic, anhydrous | USP | 35.08 |
| Microcrystalline Cellulose (Avicel® PH102; FMC Corporation, Philadelphia, PA) | NF | 56.12 |
| Stodium starch glycolate (Explotab; Penwalt, Patterson, NJ) | NF | 5.00 |
| Magnesium stearate | NF | 1.50 |

| | | |
|---|---|---|
| ^{(a)} Based on a theoretical active compound substance potency of 77.1%. | | |

| 3 mg immediate release tablet | | |
|---|---|---|
| Component | Grade of Component | Weight (mg/tablet) |
| Active compound | Pharmaceutical | 3.89^{(a)} |
| Calcium phosphate dibasic, anhydrous | USP | 34.79 |
| Microcrystalline Cellulose (Avicel® PH102) | NF | 54.82 |
| Stodium starch glycolate (Explotab) | NF | 5.00 |
| Magnesium stearate | NF | 1.50 |

| | | |
|---|---|---|
| ^{(a)} Based on a theoretical active compound substance potency of 77.1%. | | |

| 3 mg controlled release tablet | | |
|---|---|---|
| Component | Grade of Component | Weight (mg/tablet) |
| Active compound | Pharmaceutical | 3.89^{(a)} |
| Mannitol 2080, granular form | USP | 34.00 |
| Fumaric acid | NF | 12.00 |
| Microcrystalline cellulose | NF | 48.61 |
| Magnesuim Stearate (1st addition) | NF | 0.50 |
| Magnesuim Stearate (2nd addition) | NF | 1.00 |
| Cellulose Acetate (CA-398-10) Eastman Chemical, Kingsport, TX | NF | 11.90 |
| Polyethylene glycol (Carbowax PEG 3350; Union Carbide, Charleston, WV | NF | 5.10 |
| Purified Water^{(b)} | USP | (35.70) |
| Acetone^{(b)} | NF | (117.30) |

| | | |
|---|---|---|
| ^{(a)} Based on a theoretical active compound substance potency of 77.1%. | | |
| ^{(b)}The purified water and acetone are volatile and are not present in the final dosage form. | | |

| 10 mg controlled release tablet | | |
|---|---|---|
| Component | Grade of Component | Weight (mg/tablet) |
| Active compound | Pharmaceutical | 12.97^{(a)} |
| Mannitol 2080, granular form | USP | 113.32 |
| Fumaric acid | NF | 40.00 |
| Microcrystalline cellulose (Avicel PH102) | NF | 162.01 |
| Magnesuim Stearate (1st addition) | NF | 1.67 |
| Magnesuim Stearate (2nd addition) | NF | 3.33 |
| Cellulose Acetate (CA-398-10) Eastman Chemical, Kingsport, TX | NF | 33.00 |
| Polyethylene glycol (Carbowax PEG 3350) | NF | 22.00 |
| Purified Water^{(b)} | USP | (126.50) |
| Acetone^{(b)} | NF | (368.50) |

| | | |
|---|---|---|
| ^{(a)} Based on a theoretical active compound substance potency of 77.1%. | | |
| ^{(b)}The purified water and acetone are volatile and are not present in the final dosage form. In the 3 mg and 10 mg controlled release dosage forms, the active compound, mannitol, fumaric acid, microcrystalline cellulose, and magensuim stearate are tablet core components. NF is National Formulary. USP is United States Pharmacopoeia. | | |

### Preparation of 10 mg and 3 mg sustained release dosage forms

This example illustrates a method for making formulations of 3 mg and 10 mg osmotic tablets comprising a tablet core containing active compound surrounded by a semi-permeable asymmetric membrane coating. The processing of the core tablet comprised (1) blending of core components, except for magnesium stearate; (2) milling and reblending the same components; (3) adding and blending a portion of the magnesium stearate; (4) dry granulating; (5) milling/screening and reblending; (6) adding and blending the remaining portion of magnesium stearate; (7) compressing core tablets; (8) spraying an asymmetric membrane coating to the core tablets; and (9) drying.

In batch sizes of 6-14 kilograms, 2-amino-N-[2-(3aR-benzyl-2-methyl-3-oxo-2,3,3a,4,6,7-hexahydro-pyrazolo[4,3-c]pyridin-5-yl)-1R-benzyloxymethyl-2-oxo-ethyl]-isobutyramide L-tartrate was blended with all other components except magnesium stearate for 30 minutes in a suitable sized stainless steel twin-shelled blender (16 quart to 2 cubic feet). Next, the blend was passed through a mill (Fitz model JT or D mill fitted with #1A plate, medium speed, knives forward, The Fitzpatrick Company, Elmhurst, IL) and blended again for 30 minutes. Then, magnesium stearate (1st addition) was added and blended for 5 minutes. The partially lubricated blend was dry granulated using a roller compactor (Freund TF-156 roller compacter, Freund Industrial Co., Tokyo, Japan) with auger screw feed of 10-12 rpm, pressure 25 kg/cm², and roller speed of 12 rpm. The roller compactor was fitted with an oscillating roller granulator screen size of 20 mesh to mill the compacted ribbons. Next, the granulation was blended for 30 minutes before the last portion of magnesium stearate (2nd addition) was added and reblended for 5 minutes.

Using a conventional tablet press (Kilian LX21, Kilian and Co., Inc, Horsham, PA), the final blend was compressed into tablets.

A semi-permeable membrane coating (as described in U.S. patent number 5,612,059 entitled Use of Asymmetric Membranes in Delivery Devices) was applied to these tablets using a HCT-30 explosion-proof pan coater (Vector Corporation, Marion, Iowa) operated at a spray rate of 20 grams per minute, an inlet temperature of 40-45°C and air flow rate of 30 cfm (14158.43 cm³/sec). The asymmetric membrane coating formulations applied to the 3 mg and 10 mg core tablet released 80% of the dose in 10-12 hours in simulated gastric fluid (sgn) at pH about 1.2, which procedure is well known and disclosed in USPXXIII. The coating for the 3 mg tablets consisted of cellulose acetate/polyethylene glycol/water/acetone ratios of 7/3/21/69 (w/w) coated to an increase in the initial weight of 17 weight%. Likewise, for the 10 mg tablet, the coating was composed of 6/4/23/67 formulation applied to 15.5 weight%. The coated tablets were dried in the coater for 15 minutes at an inlet temperature set point of 60°C and dried in an oven (Gruenberg solvent tray oven, Gruenberg Oven Company, Williamsport, PA) for 16 hours at 50°C before testing for dissolution performance. After drying, the weight of the applied coating material was determined based on a percentage of the initial core tablet weight.

### Preparation of 1 mg and 3 mg immediate release dosage forms

1. Blend calcium phosphate dibasic anhydrous, microcystalline cellulose, and sodium starch glycolate for 5 minutes in an amber glass bottle using a Turbula mixer (20 rpm setting).
2. Screen the excipient mixture from step 1 through a 40 mesh screen and blend for 15 minutes in an amber glass bottle using a Turbula mixer (20 rpm setting)(WAB, Basel, Switzerland).
3. Add growth hormone secretagogue to the excipient mixture from step 2 using geometric dilution. After each dilution, blend for 10 minutes in an amber glass bottle using a Turbula mixer (20 rpm setting).
4. Screen this active blend from step 3 through a 40 mesh screen and blend for 10, 20 and 30 minutes in an amber glass bottle using a Turbula mixer (20 rpm setting). Remove top, middle and bottom samples at each time point.
5. Add 1.0% (before granulation) magnesium stearate and blend for 5 minutes in an amber glass bottle using a Turbula mixer (20 rpm setting).
6. Roller compact the blend using the TF Freund mini roller compactor,
   roll pressure: 40 kg/cm²
   roll speed: 3 rpm
   feed speed: 10 rpm
7. Mill the compacts using the rotating granulator fitted with a 30 mesh screen.
8. Add 0.5% (after granulation) magnesium stearate to the active granulation and blend for 5 minutes in an amber glass bottle using a Turbula mixer (20 rpm setting).
9. Tablet using a single station press (F-Press, Manesty Machines, Liverpool, England).

### Alternative preparation of 1 mg and 3 mg immediate release dosage forms

1. Blend calcium phosphate dibasic anhydrous, microcrystalline cellulose, and sodium starch glycolate in a 4-quart V-blender for 15 minutes.
2. Discharge blender.
3. Add growth hormone secretagogue and an approximately equal volume of excipient blend from step 2 to an amber glass bottle and blend for 15 minutes using a Turbula mixer (20 rpm setting).
4. Place approximately % of the excipient blend from step 2 into the V-blender.
5. Pass drug/excipient blend from step 3 through a 40 mesh screen and place in V-blender. Use a mortar and pestle to reduce the size of any agglomerates that do not pass through the screen.
6. Place the remaining excipient blend from step 2 into the V-blender.
7. Blend in the V-blender for 15 minutes.
8. Pass the blend from step 7 through a Fitzpatrick JT mill fitted with a #1 plate with knives forward and at medium speed (The Fitzpatrick Company, Elmhurst, IL).
9. Place the blend from step 8 in the 4-quart V-blender and blend for 15 minutes.
10. Add magnesium stearate (1.0% before granulation) and blend for 5 minutes.
11. Roller compact the blend using a Freund TF-Mini Roller compactor,
   roll pressure: 40 kg/cm²
   feed speed:12 rpm
   roll speed: 3 rpm
12. Mill the compacts from step 11 using the rotating granulator fitted with a 30 mesh screen.
13. Place the active granulation from step 12 in the 4-quart V-blender and blend for 10 minutes.
14. Add 0.5% (after granulation) magnesium stearate and blend for 15 minutes.
15. Tablet the blend using the Kilian T100 rotary press (Kilan and Company, Horsham, PA).

## Claims

1. A method of administering a growth hormone secretagogue to a patient, the method comprising the step of intermittently administering to a patient in need of growth hormone secretagogue administration a therapeutically effective amount of a growth hormone secretagogue.

2. A method according to Claim 1 wherein the growth hormone secretagogue is administered every second day, every third day, every fourth day, or every fifth day.

3. A method according to Claim 1 wherein the growth hormone secretagogue is administered three times a week or four times a week.

4. A method according to any of Claims 1 to 3 wherein the growth hormone secretagogue is administered orally in a tablet or capsule.

5. A method according to any of Claims 1 to 3 wherein the growth hormone secretagogue is administered using an immediate release formulation or a controlled release formulation.

6. A method according to any of Claims 1 to 3 wherein the growth hormone secretagogue is administered using a combination of immediate release and controlled release formulations.

7. A method according to any of Claims 1 to 6 wherein the growth hormone secretagogue is 2-amino-N-{1-(R)-(2,4-difluorobenzyloxymethyl)-2-oxo-2-[3-oxo-3a-(R)-pyridin-2-ylmethyl-2-(2,2,2-trifluoroethyl)-2,3,3a,4,6,7-hexahydro-pyrazolo[4,3-c]pyridin-5-yl]ethyl}-2-methylpropionamide or a pharmaceutically acceptable salt or prodrug thereof, or a salt of the prodrug.

8. A method according to Claim 7 wherein the growth hormone secretagogue is the (L)-(+)-tartaric acid salt of 2-amino-N-{1-(R)-(2,4-difluorobenzyloxymethyl)--2-oxo-2-[3-oxo-3a-(R)-pyridin-2-ylmethyl-2-(2,2,2-trifluoroethyl)-2,3,3a,4,6,7-hexahydropyrazolo[4,3-c]pyridin-5-yl]ethyl}-2-methylpropionamide.

9. A method according to any of Claims 1 to 6 wherein the growth hormone secretagogue is 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]-isobutyramide or a pharmaceutically acceptable salt or prodrug thereof, or a salt of the prodrug.

10. A method according to Claim 9 wherein the growth hormone secretagogue is the L-tartrate salt of 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]-isobutyramide, that is, 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]isobutyramide L-tartrate.

11. A method according to Claim 10 wherein 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]isobutyramide L-tartrate is administered every second day.

12. A method according to Claim 10 wherein 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]isobutyramide L-tartrate is administered every third day.

13. A method according to any of Claims 10 to 12 wherein the 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]isobutyramide L-tartrate is administered orally in a tablet or capsule.

14. A method according to any of Claims 10 to 13 wherein 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]isobutyramide L-tartrate is administered in an amount in the range of about 1 mg to about 10 mg per day on the days of administration.

15. A method according to any of Claims 10 to 14 wherein the intermittent administration of 2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxoethyl]-isobutyramide L-tartrate results in a peak plasma concentration in humans of growth hormone of about 5ng/ml or greater.

16. A method according to any of Claims 1 to 15 wherein the patient is obese or has musculoskeletal frailty, osteoporosis, congestive heart failure or insulin resistance.

17. A kit which comprises:
a. a composition comprising a growth hormone secretagogue;
b. a package for containing the composition; and
c. instructions for intermittently administering the composition comprising the growth hormone secretagogue.

18. A kit according to Claim 17 which further comprises, in addition to a composition comprising a growth hormone secretagogue, an additional composition comprising a second pharmaceutical compound which is suitable for treating obesity, musculoskeletal frailty, osteoporosis, congestive heart failure or insulin resistance.

19. A kit according to Claim 17 or 18 wherein the composition comprising a growth hormone secretagogue is in the form of a tablet or capsule.

20. A kit according to any of Claims 17 or 19 wherein the growth hormone secretagogue is
2-amino-N-{1-(R)-(2,4-difluorobenzyloxymethyl)-2-oxo-2-[3-oxo-3a-(R)-pyridin-2-ylmethyl-2-(2,2,2-trifluoroethyl)-2,3,3a,4,6,7-hexahydropyrazolo-[4,3-c]pyridin-5-yl]ethyl}-2-methylpropionamide;
(L)-(+)-tartaric acid salt of 2-amino-N-{1-(R)-(2,4-difluorobenzyloxymethyl)-2-oxo-2-[3-oxo-3a-(R)-pyridin-2-ylmethyl-2-(2,2,2-trifluoroethyl)-2,3,3a,4,6,7-hexahydropyrazolo[4,3-c]pyridin-5-yl]ethyl}-2-methylpropionamide;
2-amino-N-[2-(3a-(R)-benzyl-2-methyl-3-oxo-2,3,3a,4,6,7-hexahydropyrazolo-[4,3-c]pyridin-5-yl)-1-(R)-benzyloxymethyl-2-oxoethyl]isobutyramide; or
2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxoethyl]isobutyramide L-tartrate.
